# EUROPEAN PATENT APPLICATION

(11) **EP 4 644 553 A1**
(43) Date of publication of application: **05.11.2025**
(21) Application number: 23910916.8
(22) Date of filing: 29.12.2023
(51) Int. Cl.: C12N 15/62, C12N 15/113, C12N 9/22, C12N 15/10

(54) **NON-NUCLEIC ACID TARGET DETECTION METHOD BASED ON SPLIT CAS PROTEIN AND USE THEREOF**

(30) Priority: 30.12.2022 CN 202211728526
(71) Applicant: Shanghai Tolo Biotechnology Company Limited, Shanghai 200233 (CN)
(72) Inventor: WANG, Jin, Shanghai 200233 (CN); WANG, Jiaqi, Shanghai 200233 (CN); XIONG, Lei, Shanghai 200233 (CN); HU, Xiaowen, Shanghai 200233 (CN)
(74) Representative: Ipsilon
(86) International application number: PCT/CN2023/143136
(87) International publication number: WO 2024/141002

(57) **Abstract**

Provided are a non-nucleic acid target detection method based on a split Cas protein and the use thereof. Specifically, provided is a detection system for detecting a non-nucleic acid target, which detection system comprises (a) a first fusion protein or a first binding protein, and a second fusion protein or a second binding protein; (b) a Cas protein cis-cleavage substrate or a Cas protein cis-cleavage reporter; and (c) a guide RNA. In the system, a receptor can be flexibly changed according to the non-nucleic acid target, so as to achieve detection of various non-nucleic acid targets. For the first instance, a split Cas protein is used to detect a non-nucleic acid target, which has the characteristics of high levels of sensitivity, strong specificity and a short consumption time.

## Description

### Technical field

The present invention relates to the field of biotechnology, and specifically, it relates to a non-nucleic acid target detection method based on a split Cas protein and the use thereof.

### Background

CRISPR/Cas (Clustered regularly interspaced short palindromic repeats, CRISPRs) is an adaptive immune system in bacteria. Cas proteins target and degrade foreign nucleic acids through RNA-guided nucleases. Cas12a protein, also known as Cpf1 protein, belongs to the type V system of the second major category of CRISPR/Cas. Guided by RNA, this protein can specifically bind to and cleave exogenous DNA.

At present, a group of researchers have successfully established CRISPR/Cas12a-mediated biosensors that indicate nucleic acid content through signals such as fluorescence, color, and electrochemistry. However, transforming the existing CRISPR/Cas12a biosensing system into a more extensive detection system for non-nucleic acid analytes remains challenging, especially from the perspectives of simplicity and universality. Although other recognition elements, such as allosteric transcription factors (atf) or functional DNA molecules (DNA aptamers and DNAzymes), have been successfully integrated to achieve sensitive detection of various biological substances, including proteins, small organic molecules, bacteria, tumor cells, and virus particles, which has greatly expanded the application range of the CRISPR/Cas12a system in biosensing. However, compared with its application in the field of nucleic acid detection, the application of the CRISPR/Cas12a system in non-nucleic acid detection mainly replaces the last chromogenic reaction in traditional commercial ELISA kits. Therefore, it still has disadvantages such as a heterogeneous system, long experimental cycle, and complex operation.

Therefore, there is an urgent need in this field to develop a new method that can flexibly replace receptors according to the target to be detected, enabling the detection of various non-nucleic acid targets.

### Summary of the invention

An object of the present invention is to provide a new method that can flexibly replace receptors according to the target to be detected, enabling the detection of various non-nucleic acid targets.

Another object of the present invention is to provide a homogeneous, highly sensitive (achieving nanogram-level detection), highly specific, and rapidly visualizable (test strip detection) detection method based on split CRISPR/Cas proteins (split CRISPR/Cas) for non-nucleic acid detection targets.

Another object of the present invention is to provide a homogeneous, highly sensitive, highly specific, and rapidly visualizable detection method based on split Cas proteins for non-nucleic acid detection targets.

In the first aspect of the present invention, it provides a detection system for detecting a non-nucleic acid target, comprising:
(a1) a first fusion protein or a first binding protein, wherein the structure of the first fusion protein or the first binding protein is as set forth in Formula (I):

   Z1-L1-A1 (I);
(a2) a second fusion protein or a second binding protein, wherein the structure of the second fusion protein or the second binding protein is as set forth in Formula (II):

   A2-L2-Z2 (II);

   wherein,
   Z1 is a N-terminal fragment of a Cas protein;
   L1 is a first linker peptide;
   A1 is a first target receptor;
   A2 is a second target receptor;
   L2 is a second linker peptide;
   Z2 is a C-terminal fragment of a Cas protein;
   each "-" independently represents a linker peptide, a peptide bond, or a non-peptide bond;
(b) a Cas protein cis-cleavage substrate or a Cas protein cis-cleavage reporter; and
(c) a guide RNA, wherein the guide RNA guides the Cas protein to specifically bind to the Cas protein cis-cleavage substrate or the Cas protein cis-cleavage reporter.

In another preferred embodiment, the Cas protein cis-cleavage substrate is a component of the Cas protein cis-cleavage reporter.

In another preferred embodiment, the detection system for detecting a non-nucleic acid target further comprises:
(d) a Cas protein trans-cleavage reporter, wherein the Cas protein trans-cleavage reporter has a single-stranded nucleic acid molecule that does not hybridize with the guide sequence of the guide RNA of the Cas protein.

In another preferred embodiment, the N-terminal fragment of the Cas protein and the C-terminal fragment of the Cas protein constitute a full-length Cas protein.

In another preferred embodiment, the detection system further comprises one or more intermediate fusion proteins, wherein the structure of the intermediate fusion protein comprises an intermediate fragment of a Cas protein and target receptors located at both ends of the intermediate fragment of the Cas protein, and a linker peptide may also be included between the intermediate fragment of the Cas protein and the target receptors; wherein the N-terminal fragment of the Cas protein, the C-terminal fragment of the Cas protein, and one or more intermediate fragments of the Cas protein constitute a full-length Cas protein.

In another preferred embodiment, the detection system comprises a first detection system and a second detection system, wherein the first detection system comprises components (a1) and (a2), and the second detection system comprises components (b) and (c); preferably, the second detection system further comprises component (d).

In another preferred embodiment, the first detection system further comprises a non-nucleic acid target.

In another preferred embodiment, the Cas protein is a type II Cas protein, type V Cas protein, type VI Cas protein, or a combination thereof; or the Cas protein is a Cas protein with a RuvC structure or a HEPN structure.

In another preferred embodiment, the type II Cas protein is Cas9; the type V Cas protein is Cas12; the type VI Cas protein is Cas13; or the Cas protein with a RuvC structure or a HEPN structure is Cas9, Cas12, or Cas13.

In another preferred embodiment, the type II Cas protein is Cas9.

In another preferred embodiment, the type II Cas protein is selected from the group consisting of: type II-A Cas proteins, type II-B Cas proteins, type II-C Cas proteins, type II-C variant Cas proteins, and combinations thereof.

In another preferred embodiment, the Cas9 is selected from the group consisting of: spCas9, FnCas9, SaCas9, NmCas9, St1Cas9, St3Cas9, CjCas9, and a combination thereof.

In another preferred embodiment, the Cas9 is derived from the species selected from the group consisting of: *Streptococcus pyogenes* (SpCas9), *Streptococcus thermophiles* (St1Cas9, St3Cas9), *Streptococcus mutans, Staphylococcus aureus* (SaCas9), *Campylobacter jejuni* (CjCas9), *Francisella novicida* (FnCas9), *Neisseria meningitides* (NmCas9), and a combination thereof.

In another preferred embodiment, the type V Cas protein is selected from the group consisting of: type V-A, type V-B, type V-C, type V-D, type V-E, type V-F, type V-G, type V-H, type V-I, type V-J, type V-K Cas protein, type V-L Cas protein, and a combination thereof.

In another preferred embodiment, the type V Cas protein is selected from the group consisting of: Cas12a, Cas12b, Cas12c, Cas12d, Cas12e, Cas12f, Cas12g, Cas12h, Cas12i, Cas12j, Cas12l, and a combination thereof.

In another preferred embodiment, the Cas12a is selected from the group consisting of: FnCas12a, AsCas12a, LbCas12a, Lb5Cas12a, HkCas12a, OsCas12a, TsCas12a, BbCas12a, BoCas12a, Lb4Cas12a, CeCas12a, PrCas12a, CsbCas12a, BhCas12a, SsCas12a, Lb3Cas12a, BpCas12a, PdCas12a, BfCas12a, PcCas12a, cMtCas12a, PeCas12a, LiCas12a, Lb2Cas12a, PmCas12a, MbCas12a, EeCas12a, CsbCas12a, ErCas12a, ArCas12a, BsCas12a, AbCas12a, and a combination thereof.

In another preferred embodiment, the origin of the Cas12a is selected from the group consisting of: *Leptotrichia, Listeria, Corynebacterium, Sutterella, Legionella, Treponema, Filifactor sp., Eubacterium, Streptococcus, Lactobacillus, Mycoplasma, Bacteroides sp., Flaviivola, Flavobacterium, Azospirillum, Sphaerochaeta, Gluconacetobacter, Neisseria, Rothia, Parvibaculum, Staphylococcus, Nitratifractor, Mycoplasma, Campylobacter, Lachnospiraceae,* and a combination thereof.

In another preferred embodiment, the origin of the Cas12a is selected from the group consisting of: *Francisella tularensis* (FnCas12a), *Acidaminococcus sp. BV3L6* (AsCas12a), *Lachnospiraceae bacterium ND2006* (LbCas12a), *Lachnospiraceae bacterium NC2008* (Lb5Cas12a), *Helcococcus sp kunzii* (HkCas12a), *Oribacterium sp.NK2B42* (OsCas12a), *Thiomicrospira sp.XS5* (TsCas12a), *Bacteroidales bacterium KA00251* (BbCas12a), *Bacteroidetes oral taxon 274* (BoCas12a), *Lachnospiraceae bacterium MC2017* (Lb4Cas12a), *Coprococcus eutactus* (CeCas12a), *Prevotella ruminicola strain BPI-34* (PrCas12a), *Candidatus Saccharibacteria bacterium* (CsbCas12a), *Butyrivibrio hungatei strain MB2003* (BhCas12a), *Smithella sp. SC_K08D17* (SsCas12a), *Lachnospiraceae bacterium MC2017* (Lb3Cas12a), *Bytyrivibrio proteoclasticus* (BpCas12a), *Prevotella disens* (PdCas12a), *Butyrivibrio fibrisolvens MD2001* (BfCas12a), *Porphyromonas crevioricanis* (PcCas12a), *Candidatus Methanoplasma termitum* (CMtCas12a), *Peregrinibacteria bacterium* (PeCas12a), *Leptospira inadaiserovar Lyme* (LiCas12a), *Lachnospiraceae bacterium MA2020* (Lb2Cas12a), *Porphyromonas macaca* (PmCas12a), *Moraxella bovoculi 237* (MbCas12a), *Eubacterium eligens* (EeCas12a), *Candidatus Saccharibacteria bacterium* (CsbCas12a), *Eubacte riumrectale* (ErCas12a), *Agathobacter rectalisstrain* (ArCas12a), *Butyrivibrio sp. NC3005* (BsCas12a), *Arcobacter butzleri* (AbCas12a), and a combination thereof.

In another preferred embodiment, the origin of the Cas12b is selected from the group consisting of: *Alicyclobacillus kakegawensis,* V3-13 species of *Bacillus sp., Bacillus hisashii, Lentisphaeria bacterium, Laceyella sediminis,* and a combination thereof.

In another preferred embodiment, the Cas12b is selected from the group consisting of: AacCas12b, AaCas12b, BthCas12b, AapCas12b, AkCas12b, AmCas12b, Bs3Cas12b, LsCas12b, and a combination thereof.

In another preferred embodiment, the type VI Cas protein is Cas13.

In another preferred embodiment, the type VI Cas protein is selected from the group consisting of: type VI-A Cas proteins, type VI-B Cas proteins, type VI-D Cas proteins, type VI-X Cas proteins, type VI-Y Cas proteins, and combinations thereof.

In another preferred embodiment, the type VI Cas protein is selected from the group consisting of: Cas13a, Cas13b, Cas13c, Cas13d, Cas13e, Cas13f, Cas13x, Cas13y, and a combination thereof.

In some embodiments, the Cas13 protein is or is derived from the species selected from the group consisting of: *Alistipes, Anaerosalibacter, Bacteroides, Bacteroidetes, Bergeyella, Blautia, Butyrivibrio, Capnocytophaga, Carnobacterium, Chloroflexus, Chryseobacterium, Clostridium, Demequina, Eubacteriaceae, Eubacterium, Flavobacterium, Fusobacterium, Herbinix, Insolitispirillum, Lachnospiraceae, Leptotrichia, Listeria, Myroides, Paludibacter, Phaeodactylibacter, Porphyromonadaceae, Porphyromonas, Prevotella, Pseudobutyrivibrio, Psychroflexus, Reichenbachiella, Rhodobacter, Riemerella, Sinomicrobium, Thalassospira, Ruminococcus;* preferably, *Leptotrichia shahii, Listeria seeligeri, Lachnospiraceae bacterium* (e.g., Lb MA2020, Lb NK4A179, Lb NK4A144), *Clostridium aminophilum* (e.g., CaDSM 10710), *Carnobacterium gallinarum* (e.g., Cg DSM 4847), *Paludibacter propionicigenes* (e.g., Pp WB4), *Listeria weihenstephanensis* (e.g., Lw FSL R9-0317), *Listeriaceae bacterium* (e.g., Lb FSL M6-0635), *Leptotrichia wadei* (e.g., Lw F0279), *Rhodobacter capsulatus* (e.g., Rc SB 1003, Rc R121, Rc DE442), *Leptotrichia buccalis* (e.g., Lb C-1013-b), *Herbinix hemicellulosilytica, Eubacteriaceae bacterium* (e.g., Eb CHKCI004), *Blautia sp.* Marseille-P2398, *Leptotrichia sp.* oral taxon 879 str. F0557, *Chloroflexus aggregans, Demequina aurantiaca, Thalassospira sp.* TSL5-1, *Pseudobutyrivibrio sp.* OR37, *Butyrivibrio sp.* YAB3001, *Leptotrichia sp.* Marseille-P3007, *Bacteroides ihuae, Porphyromonadaceae bacterium* (e.g., PbKH3CP3RA), *Listeria riparia, Insolitispirillum peregrinum, Alistipes sp.* ZOR0009, *Bacteroides pyogenes* (e.g., Bp F0041), *Bacteroidetes bacterium* (e.g., Bb GWA2_31_9), *Bergeyella zoohelcum* (e.g., Bz ATCC 43767), *Capnocytophaga canimorsus, Capnocytophaga cynodegmi, Chryseobacterium carnipullorum, Chryseobacterium jejuense, Chryseobacterium ureilyticum, Flavobacterium branchiophilum, Flavobacterium columnare, Flavobacterium sp.* 316, *Myroides odoratimimus* (e.g., Mo CCUG 10230, Mo CCUG 12901, Mo CCUG 3837), *Paludibacter propionicigenes, Phaeodactylibacter xiamenensis, Porphyromonas gingivalis* (e.g., Pg F0185, Pg F0568, Pg JCVI SC001, Pg W4087), *Porphyromonas gulae, Porphyromonas sp.* COT-052OH4946, *Prevotella aurantiaca, Prevotella buccae* (e.g., Pb ATCC 33574), *Prevotella falsenii, Prevotella intermedia* (e.g., Pi 17, Pi ZT), *Prevotella pallens* (e.g., Pp ATCC 700821), *Prevotella pleuritidis, Prevotella saccharolytica* (e.g., Ps F0055), *Prevotella sp.* MA2016, *Prevotella sp.* MSX73, *Prevotella sp.* P4-76, *Prevotella sp.* P5-119, *Prevotella sp.* P5-125, *Prevotella sp.* P5-60, *Psychroflexus torquis, Reichenbachiella agariperforans, Riemerella anatipestifer, Sinomicrobium oceani, Fusobacterium necrophorum* (e.g., Fn subsp. funduliforme ATCC 51357, FnDJ-2, Fn BFTR-1, Fn subsp. Funduliforme), *Fusobacterium perfoetens* (e.g., Fp ATCC 29250), *Fusobacterium ulcerans* (e.g., Fu ATCC 49185), *Anaerosalibacter sp.* ND1, *Eubacterium siraeum, Ruminococcus flavefaciens* (e.g., Rfx XPD3002), *Ruminococcus albus,* and a combination thereof.

In another preferred embodiment, the Cas13a protein is selected from the group consisting of: LwaCas13a, LbaCas13a, LshCas13a, PprCas13a, EreCas13a, LneCas13a, CamCas13a, RcaCas13a, HheCas13a, LbuCas13a, LseCas13a, LbmCas13a, LbnCas13a, RcsCas13a, RcrCas13a, RcdCas13a, CgCas13a, Cg2Cas13a, LweCas13a, LbfCas13a, Lba4Cas13a, Lba9Cas13a, LneCas13a, HheCas13a, RcaCas13a, and a combination thereof.

In another preferred embodiment, the species of the Cas13a protein is selected from the group consisting of: *Bacteroides, Blautia, Butyrivibrio, Carnobacterium, Chloroflexus, Clostridium, Demequina, Eubacterium, Herbinix, Insolitispirillum, Lachnospiraceae, Leptotrichia, Listeria, Paludibacter, Porphyromonadaceae, Pseudobutyrivibrio, Rhodobacter,* and *Thalassospira;* preferably, *Leptotrichia shahii, Listeria seeligeri, Lachnospiraceae bacterium* (e.g., Lb MA2020, Lb NK4A179, Lb NK4A144), *Clostridium aminophilum* (e.g., CaDSM 10710), *Carnobacterium gallinarum* (e.g., Cg DSM 4847), *Paludibacter propionicigenes* (e.g., Pp WB4), *Listeria weihenstephanensis* (e.g., Lw FSL R9-0317), *Listeriaceae bacterium* (e.g., Lb FSL M6-0635), *Leptotrichia wadei* (e.g., Lw F0279), *Rhodobacter capsulatus* (e.g., Rc SB 1003, Rc R121, Rc DE442), *Leptotrichia buccalis* (e.g., Lb C-1013-b), *Herbinix hemicellulosilytica, Eubacteriaceae bacterium* (e.g., Eb CHKCI004), *Blautia sp.* Marseille-P2398, *Leptotrichia sp.* oral taxon 879 str. F0557, *Chloroflexus aggregans, Demequina aurantiaca, Thalassospira sp.* TSL5-1, *Pseudobutyrivibrio sp.* OR37, *Butyrivibrio sp.* YAB3001, *Leptotrichia sp.* Marseille-P3007, *Bacteroides ihuae, Porphyromonadaceae bacterium* (e.g., PbKH3CP3RA), *Listeria riparia, Insolitispirillum peregrinum,* and a combination thereof.

In another preferred embodiment, the Cas13b is selected from the group consisting of: Cas13b-t1, Cas13b-t2, Cas13b-t3, Cas13b-t4, Cas13b-t5, Cas13b-t6, and a combination thereof.

In another preferred embodiment, the Cas13b is selected from the group consisting of: BzCas13b, PbCas13b, PspCas13b, RanCas13b, PguCas13b, PsmCas13b, CcaCas13b, AspCas13b, PauCas13b, Pin2Cas13b, Pin3Cas13b, and a combination thereof.

In another preferred embodiment, the Cas13b is or is derived from the species selected from the group consisting of: *Alistipes, Bacteroides, Bacteroidetes, Bergeyella, Capnocytophaga, Chryseobacterium, Flavobacterium, Myroides, Paludibacter, Phaeodactylibacter, Porphyromonas, Prevotella, Psychroflexus, Reichenbachiella, Riemerella,* and *Sinomicrobium;* preferably, *Alistipes sp.* ZOR0009, *Bacteroides pyogenes* (e.g., Bp F0041), *Bacteroidetes bacterium* (e.g., BbGWA2_31_9), *Bergeyella zoohelcum* (e.g., Bz ATCC 43767), *Capnocytophaga canimorsus, Capnocytophaga cynodegmi, Chryseobacterium carnipullorum, Chryseobacterium jejuense, Chryseobacterium ureilyticum, Flavobacterium branchiophilum, Flavobacterium columnare, Flavobacterium sp.* 316, *Myroides odoratimimus* (e.g., Mo CCUG 10230, Mo CCUG 12901, Mo CCUG 3837), *Paludibacter propionicigenes, Phaeodactylibacter xiamenensis, Porphyromonas gingivalis* (e.g., Pg F0185, Pg F0568, Pg JCVI SC001, Pg W4087), *Porphyromonas gulae, Porphyromonas sp.* COT-052OH4946, *Prevotella aurantiaca, Prevotella buccae* (e.g., Pb ATCC 33574), *Prevotella falsenii, Prevotella intermedia* (e.g., Pi 17, Pi ZT), *Prevotella pallens* (e.g., Pp ATCC 700821), *Prevotella pleuritidis, Prevotella saccharolytica* (e.g., Ps F0055), *Prevotella sp.* MA2016, *Prevotella sp.* MSX73, *Prevotella sp.* P4-76, *Prevotella sp.* P5-119, *Prevotella sp.* P5-125, *Prevotella sp.* P5-60, *Psychroflexus torquis, Reichenbachiella agariperforans, Riemerella anatipestifer, Sinomicrobium oceani,* and a combination thereof.

In another preferred embodiment, the Cas13c protein is or is derived from the species selected from the group consisting of: *Fusobacterium* and *Anaerosalibacter;* preferably, *Fusobacterium necrophorum* (e.g., Fn subsp. funduliforme ATCC 51357, Fn DJ-2, Fn BFTR-1, Fn subsp. Funduliforme), *Fusobacterium perfoetens* (e.g., Fp ATCC 29250), *Fusobacterium ulcerans* (e.g., Fu ATCC 49185), *Anaerosalibacter sp.* ND1, and a combination thereof.

In another preferred embodiment, the Cas13d is selected from the group consisting of: RspCas13d, RfxCas13d, EsCas13d, AdmCas13d, and a combination thereof.

In another preferred embodiment, the Cas13d is derived from the species selected from the group consisting of: *Eubacterium* and *Ruminococcus,* preferably *Eubacterium siraeum, Ruminococcus flavefaciens* (e.g., Rfx XPD3002), *Ruminococcus albus,* and a combination thereof.

In another preferred embodiment, the Cas protein comprises wild-type and mutant forms; preferably, the mutant Cas protein is the Cas12a mutant disclosed in Chinese Invention Patent Application No. CN202210508434.7.

In another preferred embodiment, the N-terminal fragment of the Cas protein comprises the Cas₁₋₇₃₀ fragment.

In another preferred embodiment, the Cas₁₋₇₃₀ fragment corresponds to the 1-730 fragment of the LbCas protein.

In another preferred embodiment, the N-terminal fragment of the Cas protein is an N-terminal fragment based on positions 1-730 of SEQ ID NO: 1.

In another preferred embodiment, the lengths of L1 and L2 are each independently 0-1000 aa, preferably 1-80 aa, more preferably 5-60 aa, and even more preferably 10-50 aa.

In another preferred embodiment, each of L1 and L2 is independently selected from the group consisting of:
(1) a polypeptide whose amino acid sequence is as set forth in SEQ ID NO: 2 or 3;
(2) a polypeptide derived from the amino acid sequence as set forth in SEQ ID NO: 2 or 3, which is formed by substituting, deleting, or adding one or several, preferably 1-20, more preferably 1-15, more preferably 1-10, more preferably 1-8, more preferably 1-3, and most preferably 1 amino acid residue and retains the function of the polypeptide described in (1).

In another preferred embodiment, the C-terminal fragment of the Cas protein comprises the Cas₇₃₁₋₁₂₂₈ fragment.

In another preferred embodiment, the Cas₇₃₁₋₁₂₂₈ fragment corresponds to the 731-1228 fragment of the LbCas protein.

In another preferred embodiment, the C-terminal fragment of the Cas protein is a C-terminal fragment based on positions 731-1228 of SEQ ID NO: 1.

In another preferred embodiment, the N-terminal fragment of the Cas protein and the C-terminal fragment of the Cas protein together constitute a Cas protein with cis and trans cleavage activity.

In another preferred embodiment, the first target receptor and the second target receptor each independently bind specifically to the non-nucleic acid target.

In another preferred embodiment, the first target receptor and the second target receptor are each independently selected from the group consisting of: amino acid receptors, non-amino acid receptors, and combinations thereof.

In another preferred embodiment, the first target receptor and the second target receptor are each independently selected from the group consisting of: polypeptide receptors, nucleic acid aptamers, and combinations thereof; preferably, the first target receptor and the second target receptor are polypeptide receptors, and preferably, the first target receptor and the second target receptor are antibodies.

In another preferred embodiment, the non-nucleic acid target is selected from the group consisting of: proteins, polypeptides, glycoproteins, lipids, lipoproteins, cell membrane surface receptors, viruses, bacteria, archaea, fungi, antibodies, metabolites, pathogens, medicament residues, toxins, pollutants, poisons, small molecule compounds, polymers, metal ions, metal salts, small organic compounds, whole cells, and combinations thereof.

In another preferred embodiment, the non-nucleic acid target includes but is not limited to lipids, carbohydrates, peptides, proteins, glycoproteins (N-linked or O-linked), lipoproteins, phosphoproteins, specific phosphorylated or acetylated variants of proteins, amidated variants of proteins, hydroxylated variants of proteins, methylated variants of proteins, ubiquitinated variants of proteins, sulfated variants of proteins, viral capsid proteins, extracellular proteins, intracellular proteins, antibodies, and antigen-binding fragments. In another preferred embodiment, the non-nucleic acid target is selected from the group consisting of: rapamycin or an analog thereof, abscisic acid, and a combination thereof.

In another preferred embodiment, the rapamycin or an analog thereof comprises rapamycin, deforolimus, and everolimus.

In another preferred embodiment, the non-nucleic acid target is selected from the group consisting of: medicament residues, pesticide residues, farm chemical residues, organic pollutants, biomarkers, and combinations thereof.

In another preferred embodiment, the medicament residues are selected from the group consisting of: penicillins, cephalosporins, tetracyclines, sulfonamides, aminoglycosides, aminocyclitols, macrolides, quinolones, ionophores, carbadox, nitrofuran antibiotics, chloramphenicols, and mixtures thereof.

In another preferred embodiment, the medicament residue is an antibiotic medicament residue, and the antibiotic medicament residue is a residue of an antibiotic selected from the group consisting of: β-lactam antibiotics, sulfonamide antibiotics, tetracycline antibiotics, aminoglycoside antibiotics, macrolide antibiotics, and chloramphenicol antibiotics.

In another preferred embodiment, the β-lactam antibiotics are selected from the group consisting of: penicillin, cephalosporin, ceftiofur, ampicillin, amoxycillin, cloxacillin, and oxacillin.

In another preferred embodiment, the sulfonamide antibiotics are selected from the group consisting of: sulfadimidine, sulfadimethoxine, sulfamerazine, sulfathiazole, and sulfadiazine.

In another preferred embodiment, the tetracycline antibiotics are selected from the group consisting of: tetracycline, chlortetracycline, oxytetracycline, and doxycycline (deoxytetracycline).

In another preferred embodiment, the aminoglycoside antibiotics are selected from the group consisting of: gentamicin, neomycin, streptomycin, and dihydrostreptomycin.

In another preferred embodiment, the macrolide antibiotics are selected from the group consisting of: erythromycin, tylosin, and lincomycin.

In another preferred embodiment, the chloramphenicol antibiotics are selected from the group consisting of: chloramphenicol, thiamphenicol, and florfenicol.

In another preferred embodiment, the medicament residues are selected from the group consisting of: ampicillin, kanamycin, streptomycin, tetracycline, oxytetracycline, penicillin G, enrofloxacin, diclofenac, sulfadimethoxine, lincomycin, diethylstilbestrol, and a combination thereof.

In another preferred embodiment, the pesticides in the pesticide residues are selected from the group consisting of: propoxur, dichlorvos, mevinphos, methacrifos, phosmet, phorate, terbufos, α-HCH, γ-HCH, β-HCH, ethion, δ-HCH, diazinon, tefluthrin, phosphamidon, methyl parathion, chlorpyrifos-methyl, heptachlor, propargite, fenchlorphos, fenitrothion, malathion, aldrin, fenthion, chlorpyrifos, disulfoton sulfoxide, parathion, dicofol, isocarbophos, terbufos sulfone, chlorfenvinphos, isofenphos, methoprene, chlordane, endosulfan, methidathion, o,p'-DDE, dieldrin, chlorfenson, profenofos, p,p'-DDE, o,p'-DDD, endrin, chlorfenapyr, o,p'-DDT, p,p'-DDD, p,p'-DDT, bioresmethrin, methoxychlor, spiromesifen, bifenthrin, fenpropathrin, cyhalothrin, mirex, acrinathrin, permethrin, pyridaben, cyfluthrin, cypermethrin, flucythrinate, etofenprox, fenvalerate, fluvalinate, deltamethrin, acetamiprid, aldicarb, aldicarb sulfone, aldicarb sulfoxide, azinphos-ethyl, buprofezin, cadusafos, carbaryl, carbofuran, 3-hydroxycarbofuran, chlorantraniliprole, clothianidin, cyantraniliprole, diflubenzuron, dimethoate, disulfoton sulfone, emamectin benzoate, ethoprophos, etoxazole, fenamiphos, fenamiphos sulfone, fenamiphos sulfoxide, fenpyroximate, fenthion sulfoxide, fipronil, fipronil desulfinyl, fipronil sulfoxide, fonofos, fipronil sulfone, flonicamid, flubendiamide, phoxim, formothion, fosthiazate, hexythiazox, imidacloprid, indoxacarb, isofenphos-methyl, methomyl, monocrotophos, pymetrozine, spinosad, thiamethoxam, mecarbam, methiocarb, methamidophos, methoxyfenozide, nitenpyram, omethoate, phorate sulfoxide, pirimicarb, pirimiphos-methyl, pyriproxyfen, rotenone, spinetoram, spirotetramat, tebufenozide, tebufenpyrad, thiacloprid, triazophos, trichlorfon, triflumuron, oxydemeton-methyl, lufenuron, chlorfluazuron, demeton and spirodiclofen.

In another preferred embodiment, the pesticide residues are selected from the group consisting of: acetamiprid, malathion, omethoate, isocarbophos, and a combination thereof.

In another preferred embodiment, the farm chemicals in the farm chemical residues are selected from the group consisting of: 2,4-D, 3-hydroxycarbofuran, acetamiprid, aldicarb sulfone, ametryn, azoxystrobin, benoxacor, bitertanol, buprofezin, cadusafos, carbendazim, carbofuran, chlorbenzuron, chlordimeform, chlorpyrifos, chlorpyrifos-methyl, cyprodinil, diazinon, dichlorvos, diclofop-methyl, dimethenamid-P, dimethomorph, diniconazole, emamectin benzoate, ethoprophos, etofenprox, fenarimol, fenitrothion, fenobucarb, fenpropathrin, fenpyroximate, fenthion, fipronil, fipronil sulfide, fipronil desulfinyl, fipronil sulfone, flufenoxuron, fluometuron, flusilazole, gibberellin, hexaconazole, hexaflumuron(diflubenzuron), indole-3-acetic acid, indole-3-butyric acid, imidacloprid, isofenphos, isomalathion, isoprocarb, isoprothiolane, malathion, metalaxyl, methidathion, metolachlor, myclobutanil, paclobutrazol, penconazole, pendimethalin, phenthoate, phorate sulfone, phosmet, phoxim, pirimicarb, prochloraz, profenofos, prometryn, pyraclostrobin, pyridaben, pyrimethanil, quinalphos, sulfotep, fluvalinate, tebuconazole, terbufos, thiabendazole, triadimefon, and triadimenol.

In another preferred embodiment, the organic pollutants are selected from the group consisting of: BPA (bisphenol A), PCB72 (2,3',5,5'-tetrachlorobiphenyl), PCB106 (2',3',4',5,5'-pentachlorobiphenyl), PCB77 (3,3',4,4'-tetrachlorobiphenyl), and a combination thereof.

In another preferred embodiment, the biomarkers are selected from the group consisting of: tumor markers, myocardial markers, cerebrovascular markers, diabetes markers, infectious disease markers, hormone markers, cytokines, thyroid hormones, drug markers, medicament residues, autoimmune markers, allergens, parasites, and combinations thereof.

In another preferred embodiment, the biomarkers are selected from the group consisting of: Testosterone, alpha fetoprotein (AFP), insulin, cardiac troponin I (cTnI), vitamin D, vitamin B12, vitamin B2, and glucose.

In another preferred embodiment, the non-nucleic acid target is rapamycin or an analog thereof, the first fusion protein has the amino acid sequence as set forth in SEQ ID NO: 4, and the second fusion protein has the amino acid sequence as set forth in SEQ ID NO: 5.

In another preferred embodiment, the non-nucleic acid target is abscisic acid, the first fusion protein has the amino acid sequence as set forth in SEQ ID NO: 6, and the second fusion protein has the amino acid sequence as set forth in SEQ ID NO: 7.

In another preferred embodiment, the first fusion protein and the second fusion protein are combined to form a complex containing the first fusion protein and the second fusion protein.

In another preferred embodiment, the Cas protein cis-cleavage substrate is selected from the group consisting of: dsDNA, ssDNA, RNA, single-stranded nucleic acid analogs, double-stranded nucleic acid analogs, and combinations thereof.

In another preferred embodiment, the Cas protein cis-cleavage substrate is dsDNA containing PAM.

In another preferred embodiment, the Cas protein cis-cleavage reporter is selected from the group consisting of: gold nanoparticle-based reporters, fluorophore-based reporters, fluorescence polarization-based reporters, colloid phase transition/dispersion-based reporters, electrochemical signal-based reporters, semiconductor signal-based reporters, and color-based reporters.

In another preferred embodiment, the Cas protein cis-cleavage reporter carries a detectable label.

In another preferred embodiment, the detectable label comprises a fluorescent group and a quenching group.

In another preferred embodiment, the fluorescent group is selected from the group consisting of: FAM, HEX, Cy3, Cy5, Cy5.5, Cy7, ROX, VIC, JOE, TET, Texas Red, FITC, LC RED640, RB200, and a combination thereof.

In another preferred embodiment, the quenching group is selected from the group consisting of: TAMARA, BHQ1, BHQ2, BHQ3, DABSYL, Dabcyl, eclipse, and a combination thereof.

In another preferred embodiment, the fluorescent group and the quenching group are each independently located at the 5' end, 3' end, and the middle part of the Cas protein cis-cleavage substrate.

In another preferred embodiment, the length of the Cas protein cis-cleavage substrate or the Cas protein cis-cleavage reporter is 18-300 nt, preferably 18-100 nt, more preferably 18-50 nt, and most preferably 18-25 nt.

In another preferred embodiment, the Cas protein cis-cleavage reporter comprises single-stranded DNA.

In another preferred embodiment, the Cas protein cis-cleavage reporter comprises single-stranded DNA with a detectable label.

In another preferred embodiment, the single-stranded DNA is fluorescent and biotin-labeled single-stranded DNA.

In another preferred embodiment, the single-stranded DNA is fluorescent-labeled single-stranded DNA.

In another preferred embodiment, the Cas protein trans-cleavage reporter is selected from the group consisting of: single-stranded nucleic acid reporters, single-stranded nucleic acid analog reporters, and combinations thereof.

In another preferred embodiment, the Cas protein trans-cleavage reporter is selected from the group consisting of: gold nanoparticle-based reporters, fluorophore-based reporters, fluorescence polarization-based reporters, colloid phase transition/dispersion-based reporters, electrochemical signal-based reporters, semiconductor signal-based reporters, color-based reporters, and combinations thereof.

In another preferred embodiment, the length of the Cas protein trans-cleavage reporter is 3-300 nt, preferably 5-100 nt, more preferably 6-50 nt, and most preferably 8-20 nt.

In another preferred embodiment, the Cas protein trans-cleavage reporter is a nucleic acid probe.

In another preferred embodiment, the nucleic acid probe carries a detectable label.

In another preferred embodiment, the detectable label comprises a fluorescent group and a quenching group.

In another preferred embodiment, the fluorescent group is selected from the group consisting of: FAM, HEX, Cy3, Cy5, Cy5.5, Cy7, ROX, VIC, JOE, TET, Texas Red, FITC, LC RED640, RB200, and a combination thereof.

In another preferred embodiment, the quenching group is selected from the group consisting of: TAMARA, BHQ1, BHQ2, BHQ3, DABSYL, Dabcyl, eclipse, and a combination thereof.

In another preferred embodiment, the fluorescent group and the quenching group are each independently located at the 5' end, 3' end, and the middle part of the nucleic acid probe.

In another preferred embodiment, the length of the nucleic acid probe is 3-300 nt, preferably 5-100 nt, more preferably 6-50 nt, and most preferably 8-20 nt.

In another preferred embodiment, the nucleic acid probe comprises single-stranded DNA.

In another preferred embodiment, the nucleic acid probe comprises single-stranded DNA with a detectable label.

In another preferred embodiment, the single-stranded DNA is fluorescent and biotin-labeled single-stranded DNA.

In another preferred embodiment, the single-stranded DNA is fluorescent-labeled single-stranded DNA.

In another preferred embodiment, the single-stranded DNA is a fluorescent probe labeled with the fluorescent group 6-FAM at the 5' end and the quenching group BHQ1 at the 3' end.

In another preferred embodiment, the length of the guide RNA is 20-28 nt, preferably 20-25 nt, and more preferably 20-23 nt.

In another preferred embodiment, the detection includes: qualitative detection or quantitative detection.

In another preferred embodiment, the detection system further comprises (e) a buffer.

In another preferred embodiment, the detection system further comprises a non-nucleic acid target to be detected.

In another preferred embodiment, the concentration of the non-nucleic acid target to be detected in the detection system is 0.1 pM-100 mM; preferably, 0.01 nM-500 nM; more preferably, 0.02 nM-500 nM; and more preferably, 0.03 nM-500 nM.

In another preferred embodiment, the concentration of the first fusion protein or the first binding protein in the detection system is 0.01 µM-1 µM.

In another preferred embodiment, the concentration of the second fusion protein or the second binding protein in the detection system is 0.01 µM-1 µM.

In another preferred embodiment, the concentration of the guide RNA in the detection system is 0.005 µM-0.5 µM.

In another preferred embodiment, the concentration of the cis-cleavage substrate or the cis-cleavage reporter in the detection system is 0.05 µM-0.5 µM.

In another preferred embodiment, the concentration of the trans-cleavage reporter is 0.25 µM-2.5 µM.

In another preferred embodiment, the molar ratio of the first fusion protein or the first binding protein, the second fusion protein or the second binding protein, the guide RNA, the cis-cleavage substrate or the cis-cleavage reporter, and the trans-cleavage reporter is 0.5-2: 0.5-2: 0.25-1: 2.5-10: 12.5-50.

In another preferred embodiment, the pH of the detection system is 6.2-9.1, preferably 7.4-8.5.

In the second aspect of the present invention, it provides a kit for detecting a non-nucleic acid target, comprising:
i) a first container and a first fusion protein or a first binding protein, and a second fusion protein or a second binding protein located within the first container; the structure of the first fusion protein or the first binding protein is as set forth in Formula I:

   Z1-L1-A1 (I);

   the structure of the second fusion protein or the second binding protein is as set forth in Formula II:

   A2-L2-Z2 (II);

   wherein,
   Z1 is a N-terminal fragment of a Cas protein;
   L1 is a first linker peptide;
   A1 is a first target receptor;
   A2 is a second target receptor;
   L2 is a second linker peptide;
   Z2 is a C-terminal fragment of a Cas protein;
   each "-" independently represents a linker peptide, a peptide bond, or a non-peptide bond;
ii) a second container and a Cas protein cis-cleavage substrate or a Cas protein cis-cleavage reporter located within the second container;
iii) an optional third container and a Cas protein trans-cleavage reporter located within the third container;
iv) a fourth container and a guide RNA located within the fourth container;
v) an optional fifth container and a buffer located within the fifth container.

In another preferred embodiment, any two, three, four, or five (or all) of the first container, the second container, the third container, the fourth container, and the fifth container may be the same (or identical) or different containers.

In another preferred embodiment, when a Cas protein cis-cleavage reporter is located within the second container, the first container, the second container, and the fourth container are the same or identical container.

In another preferred embodiment, when a Cas protein cis-cleavage substrate is located within the second container, the first container, the second container, the third container, and the fourth container are the same or identical container.

In another preferred embodiment, the first container, the second container, the fourth container, and the fifth container are the same or identical container.

In another preferred embodiment, the first container, the second container, the third container, the fourth container, and the fifth container are the same or identical container.

In the third aspect of the present invention, it provides a method for detecting a non-nucleic acid target, comprising the steps of:
providing the detection system for detecting a non-nucleic acid target according to the first aspect of the present invention, wherein the detection system further comprises a sample to be detected; and detecting the signal emitted by the detection system,
wherein, if a signal emitted by the detection system is detected, it indicates that the non-nucleic acid target is present in the sample; and if no signal emitted by the detection system is detected, it indicates that the non-nucleic acid target is not present in the sample.

In another preferred embodiment, the method comprises the steps of mixing a first detection system and a second detection system, and detecting the signal emitted by the detection system; the first detection system comprises a first fusion protein or a first binding protein, a second fusion protein or a second binding protein, and a sample to be detected; the second detection system comprises a Cas protein cis-cleavage substrate, a Cas protein trans-cleavage reporter, and a guide RNA, or the second detection system comprises a Cas protein cis-cleavage reporter and a guide RNA; and the guide RNA guides the Cas protein to specifically bind to the Cas protein cis-cleavage substrate or the Cas protein cis-cleavage reporter.

In another preferred embodiment, the signal of the Cas protein cis-cleavage reporter or the Cas protein trans-cleavage reporter is selected from the group consisting of: gold nanoparticle-based detection signals, fluorophore-based detection signals, fluorescence polarization-based detection signals, colloid phase transition/dispersion-based detection signals, electrochemical-based detection signals, semiconductor-based detection signals, and combinations thereof.

In another preferred embodiment, the detection comprises fluorescence detection methods, and colloidal gold detection methods.

In another preferred embodiment, the fluorescence detection method is performed using a microplate reader or a fluorescence spectrophotometer.

In another preferred embodiment, the method is an *in vitro* method.

In another preferred embodiment, the method is non-diagnostic and non-therapeutic.

In the fourth aspect of the present invention, it provides a composition or a product combination, characterized by comprising:
(a1) a first fusion protein or a first binding protein, the structure of which is as set forth in Formula I:

   Z1-L1-A1 (I);
(a2) a second fusion protein or a second binding protein, the structure of which is as set forth in Formula II:

   A2-L2-Z2 (II);

   wherein,
   Z1 is a N-terminal fragment of a Cas protein;
   L1 is a first linker peptide;
   A1 is a first target receptor;
   A2 is a second target receptor;
   L2 is a second linker peptide;
   Z2 is a C-terminal fragment of a Cas protein;
   each "-" independently represents a linker peptide, a peptide bond, or a non-peptide bond;
(b) a Cas protein cis-cleavage substrate or a Cas protein cis-cleavage reporter; and
(c) a guide RNA, wherein the guide RNA guides the Cas protein to specifically bind to the Cas protein cis-cleavage substrate or the Cas protein cis-cleavage reporter.

In the fifth aspect of the present invention, it provides a use of a composition or a product combination, wherein the composition or the product combination is according to the fourth aspect of the present invention, characterized in that the use of the composition or the product combination is for preparing a reagent, kit or system for detecting a non-nucleic acid target, or for a method of detecting a non-nucleic acid target.

In the sixth aspect of the present invention, it provides a method for preparing a composition, product combination, reagent, kit, or system for detecting a non-nucleic acid target, characterized by comprising the steps of:
obtaining a first fusion protein or a first binding protein;
obtaining a second fusion protein or a second binding protein;
obtaining a Cas protein cis-cleavage substrate;
obtaining a guide RNA;
the structure of the first fusion protein or the first binding protein is as set forth in Formula I:

   Z1-L1-A1 (I);
the structure of the second fusion protein or the second binding protein is as set forth in Formula II:

   A2-L2-Z2 (II);

   wherein,
   Z1 is a N-terminal fragment of a Cas protein;
   L1 is a first linker peptide;
   A1 is a first target receptor;
   A2 is a second target receptor;
   L2 is a second linker peptide;
   Z2 is a C-terminal fragment of a Cas protein;
   each "-" independently represents a linker peptide, a peptide bond, or a non-peptide bond.

In another preferred embodiment, the method further comprises a step of: obtaining one or more intermediate fusion proteins or binding proteins.

In another preferred embodiment, the step of obtaining the first or second fusion protein comprises the steps of:
a1. constructing an expression plasmid for the first or second fusion protein, constructing a viral expression vector for the first or second fusion protein, constructing a chromosome integrated with the gene sequence of the first or second fusion protein, constructing a transposon containing the gene sequence of the first or second fusion protein, or constructing a phage with the gene sequence of the first or second fusion protein integrated into its genome; or constructing the DNA or mRNA of the first or second fusion protein;
a2. transforming the expression plasmid, the viral expression vector, the chromosome, the transposon, or the phage into a host; or placing the DNA or mRNA of the first or second fusion protein in a cell-free protein synthesis system;
a3. expressing and producing the first or second fusion protein;
a4. extracting the first or second fusion protein;
a5. purifying the first or second fusion protein.

In another preferred embodiment, the step of obtaining one or more intermediate fusion proteins comprises the steps of:
a1. constructing respective expression plasmids for the one or more intermediate fusion proteins, constructing respective viral expression vectors for the one or more intermediate fusion proteins, constructing respective chromosomes integrated with the gene sequences of the one or more intermediate fusion proteins, constructing respective transposons containing the gene sequences of the one or more intermediate fusion proteins, or constructing respective phages with the gene sequences of the one or more intermediate fusion proteins integrated into their genomes; or constructing respective DNA or mRNA of the one or more intermediate fusion proteins;
a2. transforming the respective expression plasmids, the respective viral expression vectors, the respective chromosomes, the respective transposons, or the respective phages into respective hosts; or placing the respective DNA or mRNA of the one or more intermediate fusion proteins in a cell-free protein synthesis system;
a3. respectively expressing and producing the one or more intermediate fusion proteins;
a4. respectively extracting the one or more intermediate fusion proteins;
a5. respectively purifying the one or more intermediate fusion proteins.

In another preferred embodiment, the host is selected from the group consisting of: prokaryotic cells, yeast cells, plant cells, insect cells, and mammalian cells.

In another preferred embodiment, the host is *Escherichia coli.*

In another preferred embodiment, the method for obtaining the Cas protein cis-cleavage substrate is chemical synthesis.

In another preferred embodiment, the method for obtaining the guide RNA is chemical synthesis.

In another preferred embodiment, the method further comprises a step of: obtaining a Cas protein trans-cleavage reporter; preferably, the method for obtaining the Cas protein trans-cleavage reporter is chemical synthesis.

In another preferred embodiment, the method further comprises a step of: obtaining a buffer.

In the seventh aspect of the present invention, it provides a use of a split protein of a Cas protein, a Cas protein cis-cleavage substrate, a Cas protein cis-cleavage reporter, a guide RNA, a Cas protein trans-cleavage reporter, or a buffer, for preparing a composition, product combination, reagent, kit, or system for detecting a non-nucleic acid target, or for a method of detecting a non-nucleic acid target.

In another preferred embodiment, the non-nucleic acid target is selected from the group consisting of: proteins, polypeptides, glycoproteins, lipids, lipoproteins, cell membrane surface receptors, viruses, bacteria, archaea, fungi, antibodies, metabolites, pathogens, medicament residues, toxins, pollutants, poisons, small molecule compounds, polymers, metal ions, metal salts, small organic compounds, whole cells, and combinations thereof.

In another preferred embodiment, the non-nucleic acid target includes but is not limited to lipids, carbohydrates, peptides, proteins, glycoproteins (N-linked or O-linked), lipoproteins, phosphoproteins, specific phosphorylated or acetylated variants of proteins, amidated variants of proteins, hydroxylated variants of proteins, methylated variants of proteins, ubiquitinated variants of proteins, sulfated variants of proteins, viral capsid proteins, extracellular proteins, intracellular proteins, antibodies, and antigen-binding fragments.

In another preferred embodiment, the non-nucleic acid target is selected from the group consisting of: rapamycin or an analog thereof, abscisic acid, and a combination thereof.

In another preferred embodiment, the rapamycin or an analog thereof comprises rapamycin, deforolimus, and everolimus.

In another preferred embodiment, the non-nucleic acid target is selected from the group consisting of: medicament residues, pesticide residues, farm chemical residues, organic pollutants, biomarkers, and combinations thereof.

In another preferred embodiment, the medicament residues are selected from the group consisting of: penicillins, cephalosporins, tetracyclines, sulfonamides, aminoglycosides, aminocyclitols, macrolides, quinolones, ionophores, carbadox, nitrofuran antibiotics, chloramphenicols, and mixtures thereof.

In another preferred embodiment, the medicament residue is an antibiotic medicament residue, and the antibiotic medicament residue is a residue of an antibiotic selected from the group consisting of: β-lactam antibiotics, sulfonamide antibiotics, tetracycline antibiotics, aminoglycoside antibiotics, macrolide antibiotics, and chloramphenicol antibiotics.

In another preferred embodiment, the β-lactam antibiotics are selected from the group consisting of: penicillin, cephalosporin, ceftiofur, ampicillin, amoxycillin, cloxacillin, and oxacillin.

In another preferred embodiment, the sulfonamide antibiotics are selected from the group consisting of: sulfadimidine, sulfadimethoxine, sulfamerazine, sulfathiazole, and sulfadiazine.

In another preferred embodiment, the tetracycline antibiotics are selected from the group consisting of: tetracycline, chlortetracycline, oxytetracycline, and doxycycline (deoxytetracycline).

In another preferred embodiment, the aminoglycoside antibiotics are selected from the group consisting of: gentamicin, neomycin, streptomycin, and dihydrostreptomycin.

In another preferred embodiment, the macrolide antibiotics are selected from the group consisting of: erythromycin, tylosin, and lincomycin.

In another preferred embodiment, the chloramphenicol antibiotics are selected from the group consisting of: chloramphenicol, thiamphenicol, and florfenicol.

In another preferred embodiment, the medicament residues are selected from the group consisting of: ampicillin, kanamycin, streptomycin, tetracycline, oxytetracycline, penicillin G, enrofloxacin, diclofenac, sulfadimethoxine, lincomycin, diethylstilbestrol, and a combination thereof.

In another preferred embodiment, the pesticides in the pesticide residues are selected from the group consisting of: propoxur, dichlorvos, mevinphos, methacrifos, phosmet, phorate, terbufos, α-HCH, γ-HCH, β-HCH, ethion, δ-HCH, diazinon, tefluthrin, phosphamidon, methyl parathion, chlorpyrifos-methyl, heptachlor, propargite, fenchlorphos, fenitrothion, malathion, aldrin, fenthion, chlorpyrifos, disulfoton sulfoxide, parathion, dicofol, isocarbophos, terbufos sulfone, chlorfenvinphos, isofenphos, methoprene, chlordane, endosulfan, methidathion, o,p'-DDE, dieldrin, chlorfenson, profenofos, p,p'-DDE, o,p'-DDD, endrin, chlorfenapyr, o,p'-DDT, p,p'-DDD, p,p'-DDT, bioresmethrin, methoxychlor, spiromesifen, bifenthrin, fenpropathrin, cyhalothrin, mirex, acrinathrin, permethrin, pyridaben, cyfluthrin, cypermethrin, flucythrinate, etofenprox, fenvalerate, fluvalinate, deltamethrin, acetamiprid, aldicarb, aldicarb sulfone, aldicarb sulfoxide, azinphos-ethyl, buprofezin, cadusafos, carbaryl, carbofuran, 3-hydroxycarbofuran, chlorantraniliprole, clothianidin, cyantraniliprole, diflubenzuron, dimethoate, disulfoton sulfone, emamectin benzoate, ethoprophos, etoxazole, fenamiphos, fenamiphos sulfone, fenamiphos sulfoxide, fenpyroximate, fenthion sulfoxide, fipronil, fipronil desulfinyl, fipronil sulfoxide, fonofos, fipronil sulfone, flonicamid, flubendiamide, phoxim, formothion, fosthiazate, hexythiazox, imidacloprid, indoxacarb, isofenphos-methyl, methomyl, monocrotophos, pymetrozine, spinosad, thiamethoxam, mecarbam, methiocarb, methamidophos, methoxyfenozide, nitenpyram, omethoate, phorate sulfoxide, pirimicarb, pirimiphos-methyl, pyriproxyfen, rotenone, spinetoram, spirotetramat, tebufenozide, tebufenpyrad, thiacloprid, triazophos, trichlorfon, triflumuron, oxydemeton-methyl, lufenuron, chlorfluazuron, demeton and spirodiclofen.

In another preferred embodiment, the pesticide residues are selected from the group consisting of: acetamiprid, malathion, omethoate, isocarbophos, and a combination thereof.

In another preferred embodiment, the farm chemicals in the farm chemical residues are selected from the group consisting of: 2,4-D, 3-hydroxycarbofuran, acetamiprid, aldicarb sulfone, ametryn, azoxystrobin, benoxacor, bitertanol, buprofezin, cadusafos, carbendazim, carbofuran, chlorbenzuron, chlordimeform, chlorpyrifos, chlorpyrifos-methyl, cyprodinil, diazinon, dichlorvos, diclofop-methyl, dimethenamid-P, dimethomorph, diniconazole, emamectin benzoate, ethoprophos, etofenprox, fenarimol, fenitrothion, fenobucarb, fenpropathrin, fenpyroximate, fenthion, fipronil, fipronil sulfide, fipronil desulfinyl, fipronil sulfone, flufenoxuron, fluometuron, flusilazole, gibberellin, hexaconazole, hexaflumuron(diflubenzuron), indole-3-acetic acid, indole-3-butyric acid, imidacloprid, isofenphos, isomalathion, isoprocarb, isoprothiolane, malathion, metalaxyl, methidathion, metolachlor, myclobutanil, paclobutrazol, penconazole, pendimethalin, phenthoate, phorate sulfone, phosmet, phoxim, pirimicarb, prochloraz, profenofos, prometryn, pyraclostrobin, pyridaben, pyrimethanil, quinalphos, sulfotep, fluvalinate, tebuconazole, terbufos, thiabendazole, triadimefon, and triadimenol.

In another preferred embodiment, the organic pollutants are selected from the group consisting of: BPA (bisphenol A), PCB72 (2,3',5,5'-tetrachlorobiphenyl), PCB106 (2',3',4',5,5'-pentachlorobiphenyl), PCB77 (3,3',4,4'-tetrachlorobiphenyl), and a combination thereof.

In another preferred embodiment, the biomarkers are selected from the group consisting of: tumor markers, myocardial markers, cerebrovascular markers, diabetes markers, infectious disease markers, hormone markers, cytokines, thyroid hormones, drug markers, medicament residues, autoimmune markers, allergens, parasites, and combinations thereof.

In another preferred embodiment, the biomarkers are selected from the group consisting of: Testosterone, alpha fetoprotein (AFP), insulin, cardiac troponin I (cTnI), vitamin D, vitamin B12, vitamin B2, and glucose.

In the eighth aspect of the present invention, it provides a reagent, comprising:
a first fusion protein or a first binding protein;
a second fusion protein or a second binding protein;
a Cas protein cis-cleavage substrate or a Cas protein cis-cleavage reporter;
a guide RNA, wherein the guide RNA can guide the Cas protein to bind to the Cas protein cis-cleavage substrate; and
a buffer;
the structure of the first fusion protein or the first binding protein is as set forth in Formula I:

   Z1-L1-A1 (I);
the structure of the second fusion protein or the second binding protein is as set forth in Formula II:

   A2-L2-Z2 (II);

   wherein,
   Z1 is a N-terminal fragment of a Cas protein;
   L1 is a first linker peptide;
   A1 is a first target receptor;
   A2 is a second target receptor;
   L2 is a second linker peptide;
   Z2 is a C-terminal fragment of a Cas protein;
   each "-" independently represents a linker peptide, a peptide bond, or a non-peptide bond.

In another preferred embodiment, the N-terminal fragment of the Cas protein and the C-terminal fragment of the Cas protein constitute a full-length Cas protein.

In another preferred embodiment, the reagent further comprises:
one or more intermediate fusion proteins, wherein the structure of the intermediate fusion protein comprises an intermediate fragment of a Cas protein and target receptors located at both ends of the intermediate fragment of the Cas protein, and a linker peptide may also be included between the intermediate fragment of the Cas protein and the target receptors; wherein the N-terminal fragment of the Cas protein, the C-terminal fragment of the Cas protein, and one or more intermediate fragments of the Cas protein constitute a full-length Cas protein.

In another preferred embodiment, the reagent further comprises: a Cas protein trans-cleavage reporter.

In another preferred embodiment, the reagent further comprises: a buffer.

In the ninth aspect of the present invention, it provides a use of the reagent according to the eighth aspect of the present invention, for preparing a reagent or kit for detecting a non-nucleic acid target, or for detecting a non-nucleic acid target.

It should be understood that within the scope of the present invention, the above-mentioned technical features of the present invention and the technical features specifically described in the following (such as the examples) can be combined with each other to form a new or preferred technical solution, which is not redundantly repeated one by one herein due to space limitation.

### Description of the drawings

Figure 1 is a schematic diagram of a homogeneous non-nucleic acid target detection method based on split CRISPR/LbCas12a.
Figure 2 shows the specificity of detecting rapamycin and its analogs based on split CRISPR/LbCas12a.
Figure 3 is a fluorescence curve diagram of rapamycin concentration gradient detected based on split CRISPR/LbCas 12a.
Figure 4 shows the sensitivity of the fluorescence method for detecting rapamycin based on split CRISPR/LbCas12a.
Figure 5 shows the sensitivity of the colloidal gold method for detecting rapamycin based on split CRISPR/LbCas12a.
Figure 6 shows the specificity of detecting abscisic acid based on split CRISPR/LbCas 12a.
Figure 7 is a fluorescence curve diagram of abscisic acid concentration gradient detected based on split CRISPR/Cas12a.
Figure 8 shows the sensitivity of the fluorescence method for detecting abscisic acid based on split CRISPR/Cas12a.
Figure 9 is a graph of experimental results of pH screening of the buffer in the reaction system in Example 3.
Figure 10 is a graph of experimental results of screening of buffer additives in the reaction system in Example 3.
Figure 11 is a comparison diagram of detection results obtained by detecting rapamycin from 100 nM different types of small molecules and rapamycin respectively using the reaction system of Example 3 in Example 3.
Figure 12 is a comparison diagram of detection results obtained by detecting rapamycin from a mixture of 100 nM different types of small molecules (excluding analogs) and rapamycin using the reaction system of Example 3 in Example 3.
Figure 13 is a graph of detection results of rapamycin diluted at different ratios using the reaction system of Example 3 in Example 3, showing the sensitivity of the reaction system.
Figure 14 is a graph of detection results of rapamycin diluted at different ratios using the reaction system of Example 3 in Example 3, showing the linear range of the reaction system.
Figure 15 is a graph of detection results of rapamycin in commercial serum samples treated with different final concentrations of DMSO in the reaction system of Example 3.
Figure 16 is a graph of detection results of rapamycin in EDTA bovine whole blood samples treated with different final concentrations of EG in the reaction system of Example 3.
Figure 17 is a graph of detection results of different concentrations of rapamycin in commercial serum samples treated with the optimal final concentration of DMSO in the reaction system of Example 3, showing the sensitivity of the reaction system.
Figure 18 is a graph of detection results of different concentrations of rapamycin in commercial serum samples treated with the optimal final concentration of DMSO in the reaction system of Example 3, showing the linear range of the reaction system.
Figure 19 is a graph of detection results of rapamycin in EDTA bovine whole blood samples treated with SPR (including the optimal volume of DMSO and EG: 70% (V/V) DMSO + 30% (V/V) EG) in the reaction system of Example 3, showing the sensitivity of the reaction system.
Figure 20 is a graph of detection results of rapamycin in EDTA bovine whole blood samples treated with SPR (including the optimal volume of DMSO and EG: 70% (V/V) DMSO + 30% (V/V) EG) in the reaction system of Example 3, showing the linear range of the reaction system.

### Detailed description

After extensive and in-depth research, the inventors have developed, for the first time, a detection system capable of detecting a non-nucleic acid target, which comprises (a) a first fusion protein (when the receptor is an amino acid receptor, especially a polypeptide) or a first binding protein (when the receptor is a non-amino acid receptor), a second fusion protein (when the receptor is an amino acid receptor, especially a polypeptide) or a second binding protein (when the receptor is a non-amino acid receptor); (b) a Cas protein cis-cleavage substrate (selected when using gel electrophoresis to display results or using trans-cleavage for signal reporting) or a Cas protein cis-cleavage reporter (selected when using cis-cleavage for signal reporting); (c) a guide RNA, wherein the guide RNA guides the Cas protein to specifically bind to the Cas protein cis-cleavage substrate or the Cas protein cis-cleavage reporter; and (d) an optional Cas protein trans-cleavage reporter (selected when using trans-cleavage for signal reporting). The inventors have discovered for the first time that split Cas proteins still retain cis-cleavage activity and non-specific trans-cleavage activity on ssDNA or ssRNA after their N-terminal and C-terminal fragments are brought close to each other. The system in the specific embodiments can flexibly replace receptors according to non-nucleic acid targets, enabling the detection of various types of non-nucleic acid targets. Moreover, the specific embodiments use split Cas proteins to detect non-nucleic acid targets for the first time, which are characterized by high sensitivity, strong specificity, and short time consumption. On this basis, the inventors have completed the specific embodiments.

### Terms

CRISPR-Cas: A unique genomic element derived from bacteria and archaea, functioning as an adaptive immune defense system to resist invading phages or foreign nucleic acids. This system consists of clustered regularly interspaced short palindromic repeats (CRISPR) and CRISPR-associated proteins (referred to as Cas proteins or Cas for short).

Limit of Detection (LOD): The lowest concentration of an analyte in a sample that can be consistently detected with a typical 95% certainty probability.

The term "Cas protein" refers to the CRISPR-associated protein, which is a related protein in the CRISPR system.

The term "type II Cas protein" refers to the effector proteins of the type II family among CRISPR-associated proteins, with Cas9 being the most well-known. Cas9 is a protein with two nuclease domains (RuvC and HNH). Mediated by two RNAs, it cis-cleaves the substrate DNA to produce double-strand breaks (DSBs) with blunt ends. These two RNA molecules are crRNA and tracrRNA respectively. These two RNA molecules can be bioengineered and combined into a single guide RNA molecule (gRNA) that possesses the functions of both crRNA and tracrRNA.

The term "type V Cas protein" refers to the effector proteins of the type V family among CRISPR-associated proteins. They have diversity at the N-terminus but retain a unified RuvC-like endonuclease (RuvC) domain at the C-terminus, derived from the TnpB protein encoded by autonomous or non-autonomous transposons (Shmakov, S., Smargon, A., Scott, D., Cox, D., Pyzocha, N., Yan, W., et al. (2017).Diversity and evolution of class 2 CRISPR-Cas systems. Nat. Rev. Microbiol. 15,169-182. doi: 10.1038/nrmicro.2016.184). The type V system is further subdivided into many subtypes based on different CRISPR effector proteins.

The term "type VI Cas protein" refers to the effector proteins of the type VI family among CRISPR-associated proteins, which have RNA-mediated RNA cleavage activity.

The term "Cas12a" (formerly known as "Cpf1") refers to a crRNA-dependent endonuclease, which is an enzyme of type V-A in the CRISPR system classification.

The terms "Cas12b" and "C2c1" can be used interchangeably and refer to an sgRNA-dependent endonuclease, which is an enzyme of type V-B in the CRISPR system classification.

The term "PAM" refers to the protospacer-adjacent motif, which is a short DNA sequence directly adjacent to the DNA sequence targeted by the CRISPR effector protein. It is essential for Cas12a or Cas12b to cleave double-stranded DNA. For example, the PAM of Cas12a is TTTV, and the PAM of AacCas12b is the TTN sequence.

### Cas protein

As used herein, "Cas protein" refers to CRISPR-associated proteins (also translated in some literature as CRISPR-Cas effector proteins, CRISPR/Cas effector proteins, CRISPR-Cas effectors, or CRISPR/Cas effectors), which may be type II Cas proteins, type V Cas proteins, or type VI Cas proteins. In the specific embodiments, the Cas protein refers to a protein with cis-cleavage activity, and may also refer to a protein with both cis-cleavage activity and trans-cleavage activity, such as type V Cas protein or type VI Cas protein. Once guided by a guide RNA to bind to a cis-cleavage substrate and form a ternary complex of Cas protein-guide RNA-cis-cleavage substrate, its trans-cleavage activity can be induced, that is, it randomly cleaves single strands and their equivalents (nucleic acid equivalents such as nucleic acid analogs).

The type II Cas proteins are selected from the group consisting of: type II-A Cas proteins, type II-B Cas proteins, type II-C Cas proteins, type II-C variant Cas proteins, and combinations thereof.

The type II Cas proteins described in the specific embodiments comprise Cas9, such as spCas9, FnCas9, SaCas9, NmCas9, St1Cas9, St3Cas9, CjCas9, and a combination thereof.

The Cas9 originates from the species selected from the group consisting of: *Streptococcus pyogenes* (SpCas9), *Streptococcus thermophiles* (St1Cas9, St3Cas9), *Streptococcus mutans, Staphylococcus aureus* (SaCas9), *Campylobacter jejuni* (CjCas9), *Francisella novicida* (FnCas9), *Neisseria meningitides* (NmCas9), and a combination thereof.

The type VI Cas protein is Cas13.

The type VI Cas proteins are selected from the group consisting of: type VI-A Cas proteins, type VI-B Cas proteins, type VI-D Cas proteins, type VI-X Cas proteins, type VI-Y Cas proteins, and combinations thereof.

The type VI Cas proteins are selected from the group consisting of: Cas13a, Cas13b, Cas13c, Cas13d, Cas13e, Cas13f, Cas13x, Cas13y, and a combination thereof.

In some embodiments, the Cas13 protein is or originates from the species selected from the group consisting of: *Alistipes, Anaerosalibacter, Bacteroides, Bacteroidetes, Bergeyella, Blautia, Butyrivibrio, Capnocytophaga, Carnobacterium, Chloroflexus, Chryseobacterium, Clostridium, Demequina, Eubacteriaceae, Eubacterium, Flavobacterium, Fusobacterium, Herbinix, Insolitispirillum, Lachnospiraceae, Leptotrichia, Listeria, Myroides, Paludibacter, Phaeodactylibacter, Porphyromonadaceae, Porphyromonas, Prevotella, Pseudobutyrivibrio, Psychroflexus, Reichenbachiella, Rhodobacter, Riemerella, Sinomicrobium, Thalassospira, Ruminococcus;* preferably, *Leptotrichia shahii, Listeria seeligeri, Lachnospiraceae bacterium* (e.g., Lb MA2020, Lb NK4A179, Lb NK4A144), *Clostridium aminophilum* (e.g., CaDSM 10710), *Carnobacterium gallinarum* (e.g., Cg DSM 4847), *Paludibacter propionicigenes* (e.g., Pp WB4), *Listeria weihenstephanensis* (e.g., Lw FSL R9-0317), *Listeriaceae bacterium* (e.g., Lb FSL M6-0635), *Leptotrichia wadei* (e.g., Lw F0279), *Rhodobacter capsulatus* (e.g., Rc SB 1003, Rc R121, Rc DE442), *Leptotrichia buccalis* (e.g., Lb C-1013-b), *Herbinix hemicellulosilytica, Eubacteriaceae bacterium* (e.g., Eb CHKCI004), *Blautia sp.* Marseille-P2398, *Leptotrichia sp.* oral taxon 879 str. F0557, *Chloroflexus aggregans, Demequina aurantiaca, Thalassospira sp.* TSL5-1, *Pseudobutyrivibrio sp.* OR37, *Butyrivibrio sp.* YAB3001, *Leptotrichia sp.* Marseille-P3007, *Bacteroides ihuae, Porphyromonadaceae bacterium* (e.g., PbKH3CP3RA), *Listeria riparia, Insolitispirillum peregrinum, Alistipes sp.* ZOR0009, *Bacteroides pyogenes* (e.g., Bp F0041), *Bacteroidetes bacterium* (e.g., Bb GWA2_31_9), *Bergeyella zoohelcum* (e.g., Bz ATCC 43767), *Capnocytophaga canimorsus, Capnocytophaga cynodegmi, Chryseobacterium carnipullorum, Chryseobacterium jejuense, Chryseobacterium ureilyticum, Flavobacterium branchiophilum, Flavobacterium columnare, Flavobacterium sp.* 316, *Myroides odoratimimus* (e.g., Mo CCUG 10230, Mo CCUG 12901, Mo CCUG 3837), *Paludibacter propionicigenes, Phaeodactylibacter xiamenensis, Porphyromonas gingivalis* (e.g., Pg F0185, Pg F0568, Pg JCVI SC001, Pg W4087), *Porphyromonas gulae, Porphyromonas sp.* COT-052OH4946, *Prevotella aurantiaca, Prevotella buccae* (e.g., Pb ATCC 33574), *Prevotella falsenii, Prevotella intermedia* (e.g., Pi 17, Pi ZT), *Prevotella pallens* (e.g., Pp ATCC 700821), *Prevotella pleuritidis, Prevotella saccharolytica* (e.g., Ps F0055), *Prevotella sp.* MA2016, *Prevotella sp.* MSX73, *Prevotella sp.* P4-76, *Prevotella sp.* P5-119, *Prevotella sp.* P5-125, *Prevotella sp.* P5-60, *Psychroflexus torquis, Reichenbachiella agariperforans, Riemerella anatipestifer, Sinomicrobium oceani, Fusobacterium necrophorum* (e.g., Fn subsp. funduliforme ATCC 51357, FnDJ-2, Fn BFTR-1, Fn subsp. Funduliforme), *Fusobacterium perfoetens* (e.g., Fp ATCC 29250), *Fusobacterium ulcerans* (e.g., Fu ATCC 49185), *Anaerosalibacter sp.* ND1, *Eubacterium siraeum, Ruminococcus flavefaciens* (e.g., Rfx XPD3002), *Ruminococcus albus,* and a combination thereof.

In another preferred embodiment, the Cas13a protein is selected from the group consisting of: LwaCas13a, LbaCas13a, LshCas13a, PprCas13a, EreCas13a, LneCas13a, CamCas13a, RcaCas13a, HheCas13a, LbuCas13a, LseCas13a, LbmCas13a, LbnCas13a, RcsCas13a, RcrCas13a, RcdCas13a, CgCas13a, Cg2Cas13a, LweCas13a, LbfCas13a, Lba4Cas13a, Lba9Cas13a, LneCas13a, HheCas13a, RcaCas13a, and a combination thereof.

In another preferred embodiment, the species of the Cas13a protein is selected from the group consisting of: *Bacteroides, Blautia, Butyrivibrio, Carnobacterium, Chloroflexus, Clostridium, Demequina, Eubacterium, Herbinix, Insolitispirillum, Lachnospiraceae, Leptotrichia, Listeria, Paludibacter, Porphyromonadaceae, Pseudobutyrivibrio, Rhodobacter,* and *Thalassospira;* preferably, *Leptotrichia shahii, Listeria seeligeri, Lachnospiraceae bacterium* (e.g., Lb MA2020, Lb NK4A179, Lb NK4A144), *Clostridium aminophilum* (e.g., CaDSM 10710), *Carnobacterium gallinarum* (e.g., Cg DSM 4847), *Paludibacter propionicigenes* (e.g., Pp WB4), *Listeria weihenstephanensis* (e.g., Lw FSL R9-0317), *Listeriaceae bacterium* (e.g., Lb FSL M6-0635), *Leptotrichia wadei* (e.g., Lw F0279), *Rhodobacter capsulatus* (e.g., Rc SB 1003, Rc R121, Rc DE442), *Leptotrichia buccalis* (e.g., Lb C-1013-b), *Herbinix hemicellulosilytica, Eubacteriaceae bacterium* (e.g., Eb CHKCI004), *Blautia sp.* Marseille-P2398, *Leptotrichia sp.* oral taxon 879 str. F0557, *Chloroflexus aggregans, Demequina aurantiaca, Thalassospira sp.* TSL5-1, *Pseudobutyrivibrio sp.* OR37, *Butyrivibrio sp.* YAB3001, *Leptotrichia sp.* Marseille-P3007, *Bacteroides ihuae, Porphyromonadaceae bacterium* (e.g., PbKH3CP3RA), *Listeria riparia, Insolitispirillum peregrinum,* and a combination thereof.

In another preferred embodiment, the Cas13b is selected from the group consisting of: Cas13b-t1, Cas13b-t2, Cas13b-t3, Cas13b-t4, Cas13b-t5, Cas13b-t6, and a combination thereof.

In another preferred embodiment, the Cas13b is selected from the group consisting of: BzCas13b, PbCas13b, PspCas13b, RanCas13b, PguCas13b, PsmCas13b, CcaCas13b, AspCas13b, PauCas13b, Pin2Cas13b, Pin3Cas13b, and a combination thereof.

In another preferred embodiment, the Cas13b is or originates from the species selected from the group consisting of: *Alistipes, Bacteroides, Bacteroidetes, Bergeyella, Capnocytophaga, Chryseobacterium, Flavobacterium, Myroides, Paludibacter, Phaeodactylibacter, Porphyromonas, Prevotella, Psychroflexus, Reichenbachiella, Riemerella,* and *Sinomicrobium;* preferably, *Alistipes sp.* ZOR0009, *Bacteroides pyogenes* (e.g., Bp F0041), *Bacteroidetes bacterium* (e.g., BbGWA2_31_9), *Bergeyella zoohelcum* (e.g., Bz ATCC 43767), *Capnocytophaga canimorsus, Capnocytophaga cynodegmi, Chryseobacterium carnipullorum, Chryseobacterium jejuense, Chryseobacterium ureilyticum, Flavobacterium branchiophilum, Flavobacterium columnare, Flavobacterium sp.* 316, *Myroides odoratimimus* (e.g., Mo CCUG 10230, Mo CCUG 12901, Mo CCUG 3837), *Paludibacter propionicigenes, Phaeodactylibacter xiamenensis, Porphyromonas gingivalis* (e.g., Pg F0185, Pg F0568, Pg JCVI SC001, Pg W4087), *Porphyromonas gulae, Porphyromonas sp.* COT-052OH4946, *Prevotella aurantiaca, Prevotella buccae* (e.g., Pb ATCC 33574), *Prevotella falsenii, Prevotella intermedia* (e.g., Pi 17, Pi ZT), *Prevotella pallens* (e.g., Pp ATCC 700821), *Prevotella pleuritidis, Prevotella saccharolytica* (e.g., Ps F0055), *Prevotella sp.* MA2016, *Prevotella sp.* MSX73, *Prevotella sp.* P4-76, *Prevotella sp.* P5-119, *Prevotella sp.* P5-125, *Prevotella sp.* P5-60, *Psychroflexus torquis, Reichenbachiella agariperforans, Riemerella anatipestifer, Sinomicrobium oceani,* and a combination thereof.

In another preferred embodiment, the Cas13c protein is or originates from the species selected from the group consisting of: *Fusobacterium* and *Anaerosalibacter;* preferably, *Fusobacterium necrophorum* (e.g., Fn subsp. funduliforme ATCC 51357, Fn DJ-2, Fn BFTR-1, Fn subsp. Funduliforme), *Fusobacterium perfoetens* (e.g., Fp ATCC 29250), *Fusobacterium ulcerans* (e.g., Fu ATCC 49185), *Anaerosalibacter sp.* ND1, and a combination thereof.

In another preferred embodiment, the Cas13d is selected from the group consisting of: RspCas13d, RfxCas13d, EsCas13d, AdmCas13d, and a combination thereof.

In another preferred embodiment, the Cas13d originates from the species selected from the group consisting of: *Eubacterium* and *Ruminococcus,* preferably *Eubacterium siraeum, Ruminococcus flavefaciens* (e.g., Rfx XPD3002), *Ruminococcus albus,* and a combination thereof.

The type V Cas protein is Cas12, or the Cas protein with a RuvC structure at the C-terminus is Cas12.

In another preferred embodiment, the type V Cas protein is selected from the group consisting of: type V-A Cas protein, type V-B Cas protein, type V-C Cas protein, type V-D Cas protein, type V-E Cas protein, type V-F Cas protein, type V-G Cas protein, type V-H Cas protein, type V-I Cas protein, type V-J Cas protein, type V-K Cas protein, type V-L Cas protein, and a combination thereof;

Or, the type V Cas protein is selected from the group consisting of: Cas12a, Cas12b, Cas12c, Cas12d, Cas12e, Cas12f, Cas12g, Cas12h, Cas12i, Cas12j, Cas12l, and a combination thereof.

In another preferred embodiment, the Cas12a is selected from the group consisting of: FnCas12a, LbCas12a, ErCas12a, Evcas12a, Lb5Cas12a, HkCas12a, OsCas12a, TsCas12a, BbCas12a, BoCas12a, Lb4Cas12a, CeCas12a, PrCas12a, CsbCas12a, BhCas12a, SsCas12a, Lb3Cas12a, BpCas12a, PdCas12a, BfCas12a, PcCas12a, cMtCas12a, PeCas12a, LiCas12a, Lb2Cas12a, PmCas12a, MbCas12a, EeCas12a, CsbCas12a, ArCas12a, BsCas12a, AbCas12a, AsCas12a, and a combination thereof.

In another preferred embodiment, the origin of the Cas12a is selected from the group consisting of: *Leptotrichia, Listeria, Corynebacterium, Sutterella, Legionella, Treponema, Filifactor sp., Eubacterium, Streptococcus, Lactobacillus, Mycoplasma, Bacteroides sp., Flaviivola, Flavobacterium, Azospirillum, Sphaerochaeta, Gluconacetobacter, Neisseria, Rothia, Parvibaculum, Staphylococcus, Nitratifractor, Mycoplasma, Campylobacter, Lachnospiraceae,* and a combination thereof.

In another preferred embodiment, the origin of the Cas12a is selected from the group consisting of: *Francisella tularensis* (FnCas12a), *Acidaminococcus sp. BV3L6* (AsCas12a), *Lachnospiraceae bacterium ND2006* (LbCas12a), *Lachnospiraceae bacterium NC2008* (Lb5Cas12a), *Helcococcus sp kunzii* (HkCas12a), *Oribacterium sp.NK2B42* (OsCas12a), *Thiomicrospira sp.XS5* (TsCas12a), *Bacteroidales bacterium KA00251* (BbCas12a), *Bacteroidetes oral taxon 274* (BoCas12a), *Lachnospiraceae bacterium MC2017* (Lb4Cas12a), *Coprococcus eutactus* (CeCas12a), *Prevotella ruminicola strain BPI-34* (PrCas12a), *Candidatus Saccharibacteria bacterium* (CsbCas12a), *Butyrivibrio hungatei strain MB2003* (BhCas12a), *Smithella sp. SC_K08D17* (SsCas12a), *Lachnospiraceae bacterium MC2017* (Lb3Cas12a), *Bytyrivibrio proteoclasticus* (BpCas12a), *Prevotella disens* (PdCas12a), *Butyrivibrio fibrisolvens MD2001* (BfCas12a), *Porphyromonas crevioricanis* (PcCas12a), *Candidatus Methanoplasma termitum* (CMtCas12a), *Peregrinibacteria bacterium* (PeCas12a), *Leptospira inadaiserovar Lyme* (LiCas12a), *Lachnospiraceae bacterium MA2020* (Lb2Cas12a), *Porphyromonas macaca* (PmCas12a), *Moraxella bovoculi 237* (MbCas12a), *Eubacterium eligens* (EeCas12a), *Candidatus Saccharibacteria bacterium* (CsbCas12a), *Eubacte riumrectale* (ErCas12a), *Agathobacter rectalisstrain* (ArCas12a), *Butyrivibrio sp. NC3005* (BsCas12a), *Arcobacter butzleri* (AbCas12a), and a combination thereof.

In another preferred embodiment, the origin of the Cas12b is selected from the group consisting of: *Alicyclobacillus kakegawensis,* V3-13 species of *Bacillus sp., Bacillus hisashii, Lentisphaeria bacterium, Laceyella sediminis,* and a combination thereof.

In another preferred embodiment, the Cas12b is selected from the group consisting of: AacCas12b, AaCas12b, BthCas12b, AapCas12b, AkCas12b, AmCas12b, Bs3Cas12b, LsCas12b, and a combination thereof.

The properties of type V family effectors are shown in Table 1.

**Table 1 Properties of type V family effectors (Source: doi: 10.3389/fcell.2020.622103)**

| **Subtype** | **Marker protein** | **Length (aa)** | **PAM** | **tracrRNA/ scoutRNA** | **Processing Pre-crRNA** | **Cis-cleavage target substrate** | **Trans-cleavage target** |
|---|---|---|---|---|---|---|---|
| Type V-A | Cas12a | 1,200-1,500 | (T)TTV^{a} | None | Yes | dsDNA and ssDNA | ssDNA |
| Type V-B | Cas12b | ~1,300 | TTN | tracrRNA | No | dsDNA and ssDNA | ssDNA |
| Type V-C | Cas12c | 1,200-1,300 | TG or TN | scoutRNA | Yes | dsDNA and ssDNA | ssDNA |
| Type V-D | Cas12d | 1,200 | TA or TG | scoutRNA | Unknown | dsDNA and ssDNA | ssDNA |
| Type V-E | Cas12e | ~1,000 | TTCN | tracrRNA | Unknown | dsDNA | ssDNA |
| Type V-F | Casl4 | 400-700 | NO | tracrRNA | No | ssDNA | ssDNA |
| Type V-G | Cas12g | ~800 | NO | tracrRNA | No | ssRNA | ssRNA and ssDNA |
| Type V-H | Cas12h | ~900 | RTR^{b} | None | No | dsDNA and ssDNA | ssDNA |
| Type V-I | Cas12i | ~1,100 | TTN | None | Yes | dsDNA and ssDNA | ssDNA |
| Type V-K | Cas12k | ~650 | GTN | tracrRNA | No | dsDNA | None |
| CRISPR-Cas | Cas12j | ~750 | TBN^{c} | None | Yes | dsDNA and ssDNA | ssDNA |

| | | | | | | | |
|---|---|---|---|---|---|---|---|
| *^{a}V represents A, C, and G.* *^{b}R represents A and G.* *^{c}B represents C, G, and T* | | | | | | | |

In the embodiments, the Cas proteins referred to herein, such as Cas9, Cas12, or Cas13, also encompass functional variants of Cas proteins, or their homologs or orthologs. The Cas12a mutants disclosed in Chinese Patent Application No. CN202210508434.7 can be used in the specific embodiments. As used herein, a "functional variant" of the protein refers to a variant of such protein that at least partially retains the activity of the protein. Functional variants may include mutants (which may be insertion, deletion, or substitution mutants), including polymorphs and the like. Functional variants also include fusion products of such proteins with another generally unrelated nucleic acid, protein, polypeptide, or peptide. Functional variants can be naturally occurring or artificially created. Advantageous embodiments may involve engineered or non-naturally occurring type V DNA-targeting effector proteins.

In one embodiment, type II Cas proteins, type V Cas proteins, type VI Cas proteins, or their orthologs or homologs may contain one or more mutations, and thus the nucleic acid molecules encoding them may have one or more mutations. Mutations can be artificially introduced and may include, but are not limited to, one or more mutations in the catalytic domain.

### Fusion protein or binding protein

As used herein, the fusion proteins or binding proteins in the specific embodiments comprise a first fusion protein or a first binding protein, and a second fusion protein or a second binding protein;
wherein, the structure of the first fusion protein or the first binding protein is as set forth in Formula I:

   Z1-L1-A1 (I);
the structure of the second fusion protein or the second binding protein is as set forth in Formula II:

   A2-L2-Z2 (II);

   wherein,
   Z1 is a N-terminal fragment of a Cas protein;
   L1 is a first linker peptide;
   A1 is a first target receptor;
   A2 is a second target receptor;
   L2 is a second linker peptide;
   Z2 is a C-terminal fragment of a Cas protein;
   each "-" independently represents a linker peptide, a peptide bond, or a non-peptide bond.

In the specific embodiments, the lengths of the first and second linker peptides are independently 0-1000 aa, preferably 1-80 aa, more preferably 5-60 aa, and more preferably 10-50 aa. A preferred linker peptide is as set forth in SEQ ID NO: 2 or 3.

Preferably, the N-terminal fragment of the Cas protein and the C-terminal fragment of the Cas protein constitute a full-length Cas protein;

Or, the fusion protein further comprises one or more intermediate fusion proteins, wherein the structure of the intermediate fusion protein comprises an intermediate fragment of a Cas protein and target receptors located at both ends of the intermediate fragment of the Cas protein, and a linker peptide may also be included between the intermediate fragment of the Cas protein and the target receptors; wherein the N-terminal fragment of the Cas protein, the C-terminal fragment of the Cas protein, and one or more intermediate fragments of the Cas protein constitute a full-length Cas protein.

As used herein, the term "first fusion protein (first binding protein) or second fusion protein (second binding protein)" also includes variant forms as set forth in SEQ ID NOs: 4, 5, 6, and 7. These variant forms include (but are not limited to): deletions, insertions, and/or substitutions of 1-3 (usually 1-2, more preferably 1) amino acids, as well as addition or deletion of one or several (usually within 3, preferably within 2, more preferably within 1) amino acids at the C-terminus and/or N-terminus. For example, in the art, substitution with amino acids having similar or analogous properties usually does not alter the function of the protein. Another example is that adding or deleting one or several amino acids at the C-terminus and/or N-terminus generally does not change the structure and function of the protein. In addition, the term also includes fusion proteins in monomeric and multimeric forms. The term also includes linear as well as non-linear fusion proteins (such as cyclic peptides).

The specific embodiments also include active fragments, derivatives, and analogs of the aforementioned fusion proteins. As used herein, the terms "fragment", "derivative" and "analog" refer to polypeptides that substantially retain the function or activity of the fusion proteins of the specific embodiments. The polypeptide fragments, derivatives or analogs of the specific embodiments may be (i) polypeptides in which one or several conservative or non-conservative amino acid residues (preferably conservative amino acid residues) are substituted, or (ii) polypeptides having substituent groups in one or more amino acid residues, or (iii) polypeptides formed by fusing an antigen peptide with another compound (such as a compound that extends the half-life of the polypeptide, e.g., polyethylene glycol), or (iv) polypeptides formed by fusing additional amino acid sequences to this polypeptide sequence (fusion proteins formed by fusing with a leader sequence, a secretion sequence, or a tag sequence such as 6His). In light of the teachings herein, these fragments, derivatives, and analogs are within the scope well-known to those skilled in the art.

A preferred class of active derivatives refers to polypeptides formed by replacing at most 3, preferably at most 2, more preferably at most 1 amino acid with amino acids of similar or analogous properties, compared with the amino acid sequence of Formula I or Formula II or a combination thereof. These conservative variant polypeptides are preferably produced by amino acid substitutions according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The specific embodiments also provide analogs of the first fusion protein (or first binding protein) or the second fusion protein (or second binding protein) of the specific embodiments. The differences between these analogs and the polypeptides as set forth in SEQ ID NOs: 4, 5, 6, and 7 may lie in differences in amino acid sequences, differences in modified forms that do not affect the sequences, or a combination of both. Analogs also include analogs having residues different from natural L-amino acids (such as D-amino acids), as well as analogs having non-naturally occurring or synthetic amino acids (such as β, γ-amino acids). It should be understood that the polypeptides of the specific embodiments are not limited to the representative polypeptides exemplified above.

Modified forms (usually not altering the primary structure) include: chemically derivatized forms of polypeptides *in vivo* or *in vitro,* such as acetylation or carboxylation. Modifications also include glycosylation, such as those polypeptides produced by glycosylation modifications during the synthesis and processing of the polypeptide or in further processing steps. Such modifications can be accomplished by exposing the polypeptide to enzymes that perform glycosylation (such as mammalian glycosylases or deglycosylases). Modified forms also include sequences having phosphorylated amino acid residues (such as phosphotyrosine, phosphoserine, phosphothreonine). Also included are polypeptides that have been modified to enhance their resistance to proteolysis or to optimize their solubility properties.

In a preferred embodiment, the amino acid sequences of the first fusion protein (first binding protein) or the second fusion protein (second binding protein) of the specific embodiments are as set forth in SEQ ID NOs: 4, 5, 6, and 7.

### Guide RNA (gRNA)

As used herein, "guide RNA" refers to a guide RNA formed by the fusion of a mature crRNA with a tracrRNA, a guide RNA formed by the fusion of a mature crRNA with a scoutRNA, or a crRNA alone serving as a guide RNA.

In general, a guide RNA may comprise direct repeat sequences (also referred to as DR sequences) and a guide sequence, or essentially consist of or consist of direct repeat sequences and a guide sequence (also called a spacer in the context of the endogenous CRISPR system). Depending on the Cas protein it relies on, the gRNA in different CRISPR systems may include crRNA and tracrRNA (such as the examples using Cas12b in the specific embodiments), may also include crRNA and scoutRNA, and may also include only crRNA (such as the examples using Cas12a in the specific embodiments). The crRNA and tracrRNA can be artificially engineered and fused to form a single guide RNA (sgRNA). In some cases, the guide sequence is a polynucleotide sequence that has sufficient complementarity to the cis-cleavage substrate DNA to hybridize with the cis-cleavage substrate DNA and guide the specific binding of the CRISPR/Cas protein-guide RNA complex to the cis-cleavage substrate DNA, typically with a sequence length of 12-25 nt. The direct repeat sequences can fold into a specific structure (such as a stem-loop structure) for recognition by the Cas protein to form a complex. The guide sequence does not need to be 100% complementary to the cis-cleavage substrate DNA. The guide sequence is not complementary to the nucleic acid in the trans-cleavage reporter.

In certain embodiments, when optimally aligned, the degree of complementarity (matching degree) between the guide sequence and its corresponding cis-cleavage substrate DNA is at least 50%, at least 60%, at least 70%, at least 80%, at least 90%, at least 95%, or at least 99%. Determining the optimal alignment is within the capabilities of those of ordinary skill in the art. For example, there are publicly available and commercially available alignment algorithms and programs, such as but not limited to ClustalW, the Smith-Waterman algorithm in matlab, Bowtie, Geneious, Biopython, and SeqMan.

The crRNA described in the specific embodiments can be natural, or artificially modified, designed, and synthesized.

The terms "polynucleotide", "nucleotide sequence", "nucleic acid sequence", "nucleic acid molecule", and "nucleic acid" can be used interchangeably, and include DNA, RNA, or hybrids thereof, which can be double-stranded or single-stranded.

The term "homology" or "identity" is used to refer to the sequence matching between two polypeptides or two nucleic acids. When a position in each of the two sequences being compared is occupied by the same base or amino acid monomer subunit (e.g., a position in each of two DNA molecules is occupied by adenine, or a position in each of two polypeptides is occupied by lysine), then the molecules are identical at that position between the two sequences. Usually, the comparison is made when aligning the two sequences to achieve maximum identity. Such alignments can be performed using, for example, conventional methods for determining amino acid sequence identity. With reference to the teachings of, e.g., Smith and Waterman, 1981, Adv. Appl. Math. 2:482 Pearson&Lipman, 1988, Pro. Natl. Acad. Sci. USA85:244, Thompson et al., 1994, Nucleic Acids Res 22:467380, etc., it can be determined by running computerized algorithms (GAP, BESTFIT, FASTA, and TFASTA in the Wisconsin Genetics software package, Genetics Computer Group). It can also be determined using the BLAST algorithm available from the National Center for Biotechnology Information (NCBI, www.ncbi.nlm.nih.gov/) with default parameters.

### Cis-cleavage activity

Cis-cleavage activity refers to the activity in which a Cas protein binds to the direct repeat sequences of a guide RNA, and under the guidance of the guide sequence of the guide RNA, recognizes and binds to a nucleic acid as a substrate, forms a Cas protein-guide RNA-substrate ternary complex with the substrate, and specifically cleaves the substrate. Such substrate is called a Cas protein cis-cleavage substrate. When cis-cleavage activity is used for detection, the nucleic acid serving as the Cas protein cis-cleavage substrate is modified to have a signal reporting function; in this case, such Cas protein cis-cleavage substrate is called a Cas protein cis-cleavage reporter.

### Trans-cleavage activity

Trans-cleavage activity refers to the non-specific cleavage activity of a Cas protein on single-stranded nucleic acid molecules (which can also be equivalents of nucleic acid molecules, such as nucleic acid analog molecules) in addition to specifically cleaving the substrate after the Cas protein binds to the direct repeat sequences of a guide RNA, recognizes and binds to the substrate under the guidance of the guide sequence of the guide RNA, and forms a Cas protein-guide RNA-substrate ternary complex with the substrate. If a single-stranded nucleic acid molecule is modified to have a signal reporting function, such single-stranded nucleic acid molecule is called a trans-cleavage reporter in detection.

The name of the trans-cleavage reporter is not unified at present; it may also be referred to as a nucleic acid probe (Chinese Invention Patent Application CN 107488710 A), a detection agent DNA (Chinese Invention Patent Application CN111630163A), a masking construct (Chinese Invention Patent Application CN 115175996 A), or a single-stranded nucleic acid detector (Chinese Invention Patent Application CN111690773A). The trans-cleavage reporters in the present application should be understood in a broad sense, including nucleic acid probes, detection agent DNA, masking constructs, single-stranded nucleic acid detectors, as well as the aforementioned substances composed of equivalents of single-stranded nucleic acids (such as single-stranded nucleic acid analogs) replacing single-stranded nucleic acids.

### Detection system

The specific embodiments provide a detection system for detecting a non-nucleic acid target, comprising:
(a1) a first fusion protein or a first binding protein, wherein the structure of the first fusion protein or the first binding protein is as set forth in Formula (I):

   Z1-L1-A1 (I);
(a2) a second fusion protein or a second binding protein, wherein the structure of the second fusion protein or the second binding protein is as set forth in Formula (II):

   A2-L2-Z2 (II);

   wherein,
   Z1 is a N-terminal fragment of a Cas protein;
   L1 is a first linker peptide;
   A1 is a first target receptor;
   A2 is a second target receptor;
   L2 is a second linker peptide;
   Z2 is a C-terminal fragment of a Cas protein;
   each "-" independently represents a linker peptide, a peptide bond, or a non-peptide bond;
(b) a Cas protein cis-cleavage substrate (selected when using gel electrophoresis to display results or using trans-cleavage activity for signal reporting) or a Cas protein cis-cleavage reporter (selected when using cis-cleavage activity for signal reporting); and
(c) a guide RNA, wherein the guide RNA guides the Cas protein to specifically bind to the Cas protein cis-cleavage substrate or the Cas protein cis-cleavage reporter.
(d) an optional Cas protein trans-cleavage reporter (selected when using trans-cleavage activity for signal reporting).

Preferably, the N-terminal fragment of the Cas protein and the C-terminal fragment of the Cas protein constitute a full-length Cas protein;

Or, the fusion protein further comprises one or more intermediate fusion proteins, wherein the structure of the intermediate fusion protein comprises an intermediate fragment of a Cas protein and target receptors located at both ends of the intermediate fragment of the Cas protein, and a linker peptide may also be included between the intermediate fragment of the Cas protein and the target receptors; wherein the N-terminal fragment of the Cas protein, the C-terminal fragment of the Cas protein, and one or more intermediate fragments of the Cas protein constitute a full-length Cas protein.

In a preferred embodiment, the detection system of the specific embodiments is a split CRISPR/LbCas12a detection system, which comprises: fusion proteins of two target receptors for non-nucleic acid targets and two parts of the split CRISPR/LbCas12a protein, CRISPR/LbCas12a activator double-stranded DNA (activator dsDNA, i.e., Cas protein cis-cleavage substrate), crRNA (i.e., guide RNA), and single-stranded DNA (ssDNA) reporter (i.e., Cas protein trans-cleavage reporter).

The split CRISPR/LbCas12a protein refers to two fragments obtained by splitting the CRISPR/LbCas12a protein at the amino acid site 730 from the N-terminus. The two target receptors can be amino acid receptors or non-amino acid receptors that specifically bind to non-nucleic acid targets, such as nucleic acids and proteins, and more specifically, aptamers, polypeptides, antibodies, etc.

The crRNA is an RNA that is screened to efficiently activate the endonuclease activity of the CRISPR/LbCas 12a protein, and its sequence is as set forth in SEQ ID NO.8. The CRISPR/LbCas12a activator double-stranded DNA (activator dsDNA) is a double-stranded DNA corresponding to the crRNA, and its sequences are as set forth in SEQ ID NO.9 and SEQ ID NO.10.

The single-stranded DNA (ssDNA) reporter of the specific embodiments comprises a ssDNA FQ reporter for fluorescent detection by a microplate reader and/or a ssDNA FB reporter for detection by an immunochromatographic colloidal gold strip. The ssDNA FQ reporter is a ssDNA labeled with 6-carboxyfluorescein (6-FAM) and a fluorescence quencher (BHQ1), and the labeled product is as follows: /5'-6FAM/CCCCCCCC/3'-BHQ1/, named ssDNA FQ reporter/5'-6FAM/CCCCCCCC/3'-BHQ1/. The ssDNA FB reporter is a ssDNA labeled with 6-carboxyfluorescein (6-FAM) and biotin, and the labeled product is as follows: /5FAM/CCCCCCCC/3'-Bio/, named ssDNA DB reporter/5'-FAM/CCCCCCCC/3'-Bio/.

The preparation method of the fusion protein of the split CRISPR/LbCas12a protein and the target receptor in the specific embodiments comprises: performing prokaryotic codon optimization on the nucleic acid sequence of the fusion protein to obtain the sequence, constructing the sequence into a pET28TEV expression vector, inducing soluble protein expression at low temperature, and obtaining the target protein through affinity purification and molecular sieve purification.

The specific embodiments provide a detection method for non-nucleic acid targets based on split CRISPR/LbCas12a, which can use either fluorescent detection by a microplate reader or detection by an immunochromatographic colloidal gold strip. When using fluorescent detection by a microplate reader, the DNA (ssDNA) reporter in the CRISPR/LbCas12a detection system is the ssDNA FQ reporter; when using detection by an immunochromatographic colloidal gold strip, the DNA (ssDNA) reporter is the ssDNA FB reporter.

When using fluorescence detection by a microplate reader, in the presence of a biological target molecule to be detected in the split CRISPR/LbCas12a detection system, the biological target molecule to be detected induces dimerization between the target receptors, causing the split LbCas12a fragments to recombine and regain crRNA-guided DNA nuclease activity. The activated CRISPR/LbCas12a protein will cleave the ssDNA FQ reporter labeled with a fluorophore and a quencher, thereby releasing and activating the fluorophore. A fluorescence reading can be detected using a microplate reader. Correspondingly, when there is no target molecule in the sample to be tested, the fluorescence reading shows a baseline value.

When using immunochromatographic colloidal gold strip detection, after adding the sample to be tested, which has been cleaved by the split CRISPR/LbCas12a detection system, to the colloidal gold strip, the colloidal gold-labeled anti-FAM antibody will bind to the FAM-labeled ssDNA reporter, and the complex then moves from the control line to the test line along the direction of the liquid flow; The streptavidin-saturated control line captures the biotin-labeled ssDNA reporter, thereby displaying a band; When the split CRISPR/LbCas12a system detects a non-nucleic acid target, it will cleave the ssDNA reporter labeled with FAM and biotin, resulting in FAM-labeled ssDNA fragments being captured by the test line to produce a color. When the split CRISPR/LbCas12a system fails to detect a non-nucleic acid target, it cannot cleave the ssDNA reporter labeled with FAM and biotin, thus no FAM-labeled ssDNA fragments will be captured by the test line to produce a color.

In the examples of the specific embodiments, the Cas₁₋₇₃₀ fragment and Cas₇₃₁₋₁₂₂₈ fragment of LbCas12a are taken as examples for illustration. Among them, the Cas₁₋₇₃₀ fragment refers to the fragment at positions 1-730 of the amino acid sequence of the Cas protein based on SEQ ID NO: 1, and is also referred to as the N730 fragment of the LbCas12a protein hereinafter. The Cas₇₃₁₋₁₂₂₈ fragment refers to the fragment at positions 731-1228 of the amino acid sequence of the Cas protein based on SEQ ID NO: 1, and is also referred to as the C731 fragment of the LbCas12a protein hereinafter. The split Cas protein in each example (split Cas protein) refers to the two fragments obtained by splitting LbCas12a at the 730th amino acid site from the N-terminus. Of course, splitting can also be performed at other sites, such as 70, 86, 134, 198, 213, 248, 266, 310, 373, 406, 443, 550, 574, 607, 624, 657, 711, 762, 772, 780, 808, 824, 864, 966, 1039, 1059, 1078, 1107, 1118, 1143, 1157, 1170, 1202.

Experimental validation by the inventors has shown that when the C-terminus and N-terminus of the split Cas protein are brought into proximity, the protein retains both cis-cleavage activity and non-specific trans-cleavage activity against single-stranded nucleic acids and their equivalents. This system can flexibly replace receptors according to non-nucleic acid targets, enabling the detection of various non-nucleic acid targets. In the present invention, the receptors recognize and bind to their corresponding non-nucleic acid targets (for example, FKB and FKBP recognize and bind to rapamycin, and ABI and PYL1 recognize and bind to abscisic acid), thereby bringing the split Cas proteins close to each other. In the presence of guide RNA, cis-cleavage substrates (also referred to as "activators" herein), and trans-cleavage reporters, the split Cas proteins that are brought close together, due to their trans-cleavage activity, can cleave the trans-cleavage reporters, thereby realizing the reporting of the existence of non-nucleic acid targets. Preferably, when the C-terminus and N-terminus of the Cas protein split into two segments are brought close to each other at the split site, the protein still retains cis-cleavage activity and non-specific trans-cleavage activity against ssDNA; of course, the Cas protein can also be split into more than three segments, such as three, four, five, six, seven, eight, nine, or ten segments. In the following text, the specific embodiments take two segments as an example. As long as there are receptors that can recognize and bind to non-nucleic acid targets, the invention can be applied to detect non-nucleic acid targets.

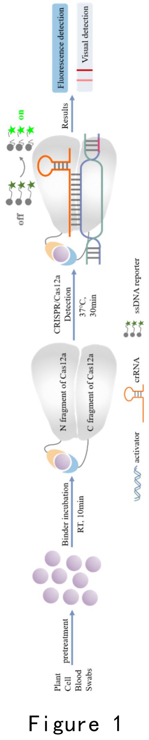

The principle of the specific embodiments is illustrated in Figure 1 and described as follows.

When using fluorescence detection by a microplate reader, in the presence of a biological target molecule to be detected in the split CRISPR/LbCas12a detection system, the biological target molecule to be detected induces dimerization between the target receptors, causing the split LbCas12a fragments to recombine and regain crRNA-guided DNA nuclease activity. The activated CRISPR/LbCas12a protein will cleave the ssDNA FQ reporter labeled with a fluorophore and a quencher, thereby releasing and activating the fluorophore. A fluorescence reading can be detected using a microplate reader. Correspondingly, when there is no target molecule in the sample to be tested, the fluorescence reading shows a baseline value.

When using immunochromatographic colloidal gold strip detection, after adding the split CRISPR/LbCas12a detection system and the sample to be tested to the colloidal gold strip, the colloidal gold-labeled anti-FAM antibody will bind to the FAM-labeled ssDNA reporter, and the complex then moves from the control line to the test line along the direction of the liquid flow; The streptavidin-saturated control line captures the biotin-labeled ssDNA reporter, thereby displaying a band; When the split CRISPR/LbCas12a system detects a non-nucleic acid target, it will cleave the ssDNA reporter labeled with FAM and biotin, resulting in FAM-labeled ssDNA fragments being captured by the test line to produce a color. When the split CRISPR/LbCas12a system fails to detect a non-nucleic acid target, it cannot cleave the ssDNA reporter labeled with FAM and biotin, thus no FAM-labeled ssDNA fragments will be captured by the test line to produce a color.

Therefore, the inventors propose a new use of the split proteins of Cas proteins, which is for preparing compositions, product combinations, reagents, kits or systems for detecting non-nucleic acid targets, or for methods of detecting non-nucleic acid targets.

The inventors also propose a new use of Cas protein cis-cleavage substrates or Cas protein cis-cleavage reporters, which is for preparing compositions, product combinations, reagents, kits or systems for detecting non-nucleic acid targets, or for methods of detecting non-nucleic acid targets. The inventors also propose a new use of guide RNAs, which is for preparing compositions, product combinations, reagents, kits or systems for detecting non-nucleic acid targets, or for methods of detecting non-nucleic acid targets. The inventors also propose a new use of Cas protein trans-cleavage reporters, which is for preparing compositions, product combinations, reagents, kits or systems for detecting non-nucleic acid targets, or for methods of detecting non-nucleic acid targets. The inventors also propose a new use of buffers, which is for preparing compositions, product combinations, reagents, kits or systems for detecting non-nucleic acid targets, or for methods of detecting non-nucleic acid targets.

The inventors also propose a composition or product combination, which is used for preparing reagents, kits or systems for detecting non-nucleic acid targets, or for methods of detecting non-nucleic acid targets. In a preferred specific embodiment, the composition or product combination comprises: a first fusion protein or a first binding protein, a second fusion protein or a second binding protein, a Cas protein cis-cleavage substrate or a Cas protein cis-cleavage reporter, and a guide RNA; further, it may also comprise a buffer. This technical solution uses the cis-cleavage activity of Cas proteins for detection, and the detection result is obtained through gel electrophoresis of the product or through the Cas protein cis-cleavage reporter. In another preferred specific embodiment, the composition comprises: a first fusion protein or a first binding protein, a second fusion protein or a second binding protein, a Cas protein cis-cleavage substrate, a guide RNA, and a Cas protein trans-cleavage reporter; further, it may also comprise a buffer. This technical solution uses the trans-cleavage activity of Cas proteins for detection, and the detection result is obtained through the Cas protein trans-cleavage reporter.

The inventors also propose a reagent, which is used for preparing kits or systems for detecting non-nucleic acid targets, or for methods of detecting non-nucleic acid targets. In a preferred specific embodiment, the reagent comprises: a first fusion protein or a first binding protein, a second fusion protein or a second binding protein, a Cas protein cis-cleavage substrate or a Cas protein cis-cleavage reporter, a guide RNA, and a buffer. This reagent uses the cis-cleavage activity of Cas proteins for detection, and the detection result is obtained through gel electrophoresis of the product or through the Cas protein cis-cleavage reporter. In another preferred specific embodiment, the reagent comprises: a first fusion protein or a first binding protein, a second fusion protein or a second binding protein, a Cas protein cis-cleavage substrate, a guide RNA, a buffer, and a Cas protein trans-cleavage reporter. This reagent uses the trans-cleavage activity of Cas proteins for detection, and the detection result is obtained through the Cas protein trans-cleavage reporter.

The inventors also propose a kit, which is used in a method for detecting non-nucleic acid targets. In a preferred specific embodiment, the kit comprises: a first container and a first fusion protein or a first binding protein, and a second fusion protein or a second binding protein located within the first container; a second container and a Cas protein cis-cleavage substrate or a Cas protein cis-cleavage reporter located within the second container; a fourth container and a guide RNA located within the fourth container; and a fifth container and a buffer located within the fifth container. This kit uses the cis-cleavage activity of Cas proteins for detection, and the detection result is obtained through gel electrophoresis of the product or through the Cas protein cis-cleavage reporter. In another preferred specific embodiment, the kit comprises: a first container and a first fusion protein or a first binding protein, and a second fusion protein or a second binding protein located within the first container; a second container and a Cas protein cis-cleavage substrate located within the second container; a third container and a Cas protein trans-cleavage reporter located within the third container; a fourth container and a guide RNA located within the fourth container; and a fifth container and a buffer located within the fifth container. This kit uses the trans-cleavage activity of Cas proteins for detection, and the detection result is obtained through the Cas protein trans-cleavage reporter.

The inventors also propose a system for detecting non-nucleic acid targets. In a preferred specific embodiment, the system for detecting non-nucleic acid targets comprises: a first fusion protein or a first binding protein, a second fusion protein or a second binding protein, a Cas protein cis-cleavage substrate or a Cas protein cis-cleavage reporter, a guide RNA, and a buffer. This system for detecting non-nucleic acid targets uses the cis-cleavage activity of Cas proteins for detection, and the detection result is obtained through gel electrophoresis of the product or through the Cas protein cis-cleavage reporter. In another preferred specific embodiment, the system for detecting non-nucleic acid targets comprises: a first fusion protein or a first binding protein, a second fusion protein or a second binding protein, a Cas protein cis-cleavage substrate, a guide RNA, a Cas protein trans-cleavage reporter, and a buffer. This kit uses the trans-cleavage activity of Cas proteins for detection, and the detection result is obtained through the Cas protein trans-cleavage reporter.

The inventors also propose a detection method based on the aforementioned composition, reagent, kit or system for detecting non-nucleic acid targets. Preferably, the method for detecting a non-nucleic acid target comprises the steps of: contacting a sample to be tested with the composition in the aforementioned preferred specific embodiment, the aforementioned reagent, the reagent in the aforementioned kit or the aforementioned detection system to obtain a first product; and performing gel electrophoresis on the first product or detecting the cis-cleavage signal of the first product. More preferably, the method for detecting a non-nucleic acid target comprises the steps of: contacting a sample to be tested with the composition, the reagent, the reagent in the kit or the detection system in another preferred specific embodiment to obtain a second product; and detecting the trans-cleavage signal of the second product.

In a preferred embodiment of the specific embodiments, in the composition, reagent, kit, system for detecting non-nucleic acid targets, and detection method, the N-terminal fragment of the Cas protein and the C-terminal fragment of the Cas protein constitute a full-length Cas protein. The N-terminal fragment of the Cas protein has 70-1170 amino acid residues; the C-terminal fragment of the Cas protein has 58-1158 amino acid residues. The first split protein of the N-terminal fragment of the Cas protein is derived from *Lachnospiraceae bacterium;* the C-terminal fragment of the Cas protein is derived from *Lachnospiraceae bacterium.*

The non-nucleic acid targets described in the specific embodiments are selected from the group consisting of: Rapamycin, also known as Sirolimus, which is a macrolide immunosuppressant suitable for patients who have received kidney transplants to prevent organ rejection; and Abscisic acid (ABA), which is an important hormone in plants, formed by the degradation of carotenoids, with the function of inhibiting the growth-promoting effects of gibberellins and cytokinins; and it is related to leaf senescence, fruit abscission, etc. Although its content is very low, it plays an important role in regulating the growth, maturation, and senescence of plants.

The Cas proteins described in the specific embodiments are type II Cas proteins, type V Cas proteins, or type VI Cas proteins; or the Cas proteins are those with RuvC domains or HEPN domains. Further, the type II Cas proteins are Cas9; the type V Cas proteins are Cas12; the type VI Cas proteins are Cas13; or the Cas proteins with RuvC domains or HEPN domains are Cas9, Cas12, or Cas13. Furthermore, the type II Cas proteins are type II-A, type II-B, or type II-C Cas proteins; the type V Cas proteins are type V-A, type V-B, type V-C, type V-D, type V-E, type V-F, type V-G, type V-H, type V-I, type V-J, type V-K, or type V-L Cas proteins; the type VI Cas proteins are type VI-A, VI-B, VI-C, VI-D, VI-X, or VI-Y. Alternatively, the type II Cas proteins are Cas9a, Cas9b, or Cas9c; the type V Cas proteins are Cas12a, Cas12b, Cas12c, Cas12d, Cas12e, Cas12f, Cas12g, Cas12h, Cas12i, Cas12j, or Cas12l proteins.

The Cas12a described in the specific embodiments is selected from the group consisting of: FnCas12a, AsCas12a, LbCas12a, Lb5Cas12a, HkCas12a, OsCas12a, TsCas12a, BbCas12a, BoCas12a, Lb4Cas12a, CeCas12a, PrCas12a, CsbCas12a, BhCas12a, SsCas12a, Lb3Cas12a, BpCas12a, PdCas12a, BfCas12a, PcCas12a, cMtCas12a, PeCas12a, LiCas12a, Lb2Cas12a, PmCas12a, MbCas12a, EeCas12a, CsbCas12a, ErCas12a, ArCas12a, BsCas12a, AbCas12a, and a combination thereof. Alternatively, the origin of the Cas12a is selected from the group consisting of: *Leptotrichia, Listeria, Corynebacterium, Sutterella, Legionella, Treponema, Filifactor sp., Eubacterium, Streptococcus, Lactobacillus, Mycoplasma, Bacteroides sp., Flaviivola, Flavobacterium, Azospirillum, Sphaerochaeta, Gluconacetobacter, Neisseria, Rothia, Parvibaculum, Staphylococcus, Nitratifractor, Mycoplasma, Campylobacter, Lachnospiraceae,* and a combination thereof. Alternatively, the origin of the Cas12a is selected from the group consisting of: *Francisella tularensis* (FnCas12a), *Acidaminococcus sp. BV3L6* (AsCas12a), *Lachnospiraceae bacterium ND2006* (LbCas12a), *Lachnospiraceae bacterium NC2008* (Lb5Cas12a), *Helcococcus sp kunzii* (HkCas12a), *Oribacterium sp.NK2B42* (OsCas12a), *Thiomicrospira sp.XS5* (TsCas12a), *Bacteroidales bacterium KA00251* (BbCas12a), *Bacteroidetes oral taxon 274* (BoCas12a), *Lachnospiraceae bacterium MC2017* (Lb4Cas12a), *Coprococcus eutactus* (CeCas12a), *Prevotella ruminicola strain BPI-*34 (PrCas12a), *Candidatus Saccharibacteria bacterium* (CsbCas12a), *Butyrivibrio hungatei strain MB2003* (BhCas12a), *Smithella sp. SC_K08D17* (SsCas12a), *Lachnospiraceae bacterium MC2017* (Lb3Cas12a), *Bytyrivibrio proteoclasticus* (BpCas12a), *Prevotella disens* (PdCas12a), *Butyrivibrio fibrisolvens MD2001* (BfCas12a), *Porphyromonas crevioricanis* (PcCas12a), *Candidatus Methanoplasma termitum* (CMtCas12a), *Peregrinibacteria bacterium* (PeCas12a), *Leptospira inadaiserovar Lyme* (LiCas12a), *Lachnospiraceae bacterium MA2020* (Lb2Cas12a), *Porphyromonas macaca* (PmCas12a), *Moraxella bovoculi 237* (MbCas12a), *Eubacterium eligens* (EeCas12a), *Candidatus Saccharibacteria bacterium* (CsbCas12a), *Eubacte riumrectale* (ErCas12a), *Agathobacter rectalisstrain* (ArCas12a), *Butyrivibrio sp. NC3005* (BsCas12a), *Arcobacter butzleri* (AbCas12a), and a combination thereof.

The origin of the Cas12b described in the specific embodiments is selected from the group consisting of: *Alicyclobacillus kakegawensis,* V3-13 species of *Bacillus sp., Bacillus hisashii, Lentisphaeria bacterium,* and *Laceyella sediminis.* Further, the Cas12b is selected from the group consisting of: AacCas12b, AaCas12b, BthCas12b, AapCas12b, AkCas12b, AmCas12b, Bs3Cas12b, and LsCas12b.

The first specific target receptor and/or the second specific target receptor described in the specific embodiments are polypeptides or nucleic acids.

The Cas protein cis-cleavage substrates described in the specific embodiments are dsDNA, ssDNA, or RNA. Specifically, the cis-cleavage substrates of Cas proteins are related to the type of Cas proteins. For example, the cis-cleavage substrates of Cas12a, Cas12b, Cas12c, Cas12d, Cas12h, Cas12i, or Cas12j can be either dsDNA or ssDNA; the cis-cleavage substrate of Cas12e is dsDNA; the cis-cleavage substrates of Cas12f and Cas12g are ssDNA.

The Cas protein trans-cleavage reporters described in the specific embodiments are selected from the group consisting of: fluorescent reporters, test strip reporters, masking construct reporters, and electrochemical sensor reporters; or the Cas protein trans-cleavage reporters are selected from gold nanoparticle-based detection reporters, fluorophore-based detection reporters, fluorescence polarization-based detection reporters, colloid phase transition/dispersion-based detection reporters, electrochemical signal-based detection reporters, semiconductor signal-based detection reporters, and color-based detection reporters.

The buffers described in the specific embodiments are selected from the group consisting of: HOLMES buffer (e.g., HOLMES 1), HOLMES Ca²⁺ buffer, NEBuffer 1.1, NEBuffer 2.1, NEBuffer 3.1, CutSmart, NEBuffer r1.1, NEBuffer r2.1, NEBuffer r3.1, NEBuffer r3.1, and rCutSmart buffer. HOLMES buffer and HOLMES Ca²⁺ buffer are purchased from Anhui Tolo Biotechnology Company Limited, and the NEBuffer series and rCutSmart buffer are purchased from New England Biolabs.

The inventors also propose a method for preparing the aforementioned composition, reagent, kit, or system for detecting non-nucleic acid targets, comprising the steps of:
obtaining a first fusion protein or a first binding protein;
obtaining a second fusion protein or a second binding protein;
obtaining a Cas protein cis-cleavage substrate; and
obtaining a guide RNA.

The step of obtaining the first or second fusion protein comprises the steps of:
a1. constructing an expression plasmid for the first or second fusion protein, constructing a viral expression vector for the first or second fusion protein, constructing a chromosome integrated with the gene sequence of the first or second fusion protein, constructing a transposon containing the gene sequence of the first or second fusion protein, or constructing a phage with the gene sequence of the first or second fusion protein integrated into its genome; or constructing the DNA or mRNA of the first or second fusion protein;
a2. transforming the expression plasmid, the viral expression vector, the chromosome, the transposon, or the phage into a host; or placing the DNA or mRNA of the first or second fusion protein in a cell-free protein synthesis system;
a3. expressing and producing the first or second fusion protein;
a4. extracting the first or second fusion protein;
a5. purifying the first or second fusion protein.

The host is selected from the group consisting of: prokaryotic cells, yeast cells, plant cells, insect cells, and mammalian cells. The host is a prokaryotic cell.

In the specific embodiments, the method for obtaining the Cas protein cis-cleavage substrate is chemical synthesis;

In the specific embodiments, the method for obtaining the guide RNA is chemical synthesis.

### The main advantages of the specific embodiments include:

(1) The specific embodiments develop for the first time a detection system capable of detecting a non-nucleic acid target, which comprises (a) a first fusion protein or a first binding protein, a second fusion protein or a second binding protein; (b) a Cas protein cis-cleavage substrate or a Cas protein cis-cleavage reporter; (c) a guide RNA, wherein the guide RNA guides the Cas protein to specifically bind to the Cas protein cis-cleavage substrate or the Cas protein cis-cleavage reporter; it may further comprise (d) a Cas protein trans-cleavage reporter. The inventors have discovered for the first time that split Cas proteins still retain cis-cleavage activity and non-specific trans-cleavage activity on ssDNA after their N-terminal and C-terminal fragments are brought close to each other. The system in the specific embodiments can flexibly replace receptors according to non-nucleic acid targets, enabling the detection of various types of non-nucleic acid targets. Moreover, the specific embodiments use split Cas proteins to detect non-nucleic acid targets for the first time, which are characterized by high sensitivity, strong specificity, and short time consumption.
(2) The specific embodiments use split CRISPR/Cas12a to detect non-nucleic acid targets for the first time, with advantages such as high sensitivity, strong specificity, and short time consumption. These advantages make the split CRISPR/Cas12a-based colloidal gold strip detection method developed in the specific embodiments convenient for rapid detection and diagnosis of biomarkers, metabolites, etc., in laboratories and clinical medicine.

The specific embodiments will be further illustrated below with reference to the specific examples. It should be understood that these examples are only to illustrate the specific embodiments, not to limit the scope of the specific embodiments. The conditions of the experimental methods not specifically indicated in the following examples are usually in accordance with conventional conditions as described in e.g., Sambrook et al., Molecular Cloning: A Laboratory Manual (New York: Cold Spring Harbor Laboratory Press, 1989), or according to the conditions recommended by the manufacturers. Percentages and parts are calculated by weight unless otherwise stated.

Unless otherwise specified, the materials and reagents used in the examples of the specific embodiments are all commercially available products.

### Example 1: Rapid and Sensitive Detection of Rapamycin and Its Derivatives

### 1.1 Protein Preparation

The gene fragments of FKB and FKBP in this example were obtained by referencing the FRB and FKBP gene segments from the NCBI database (amino acid positions 2021-2114 of accession NP_001373429.1 (corresponding to FRB) and accession NP_000792.1 (corresponding to FKBP gene)), splicing them with the N730 and C731 segments of the LbCas12a protein (SEQ ID NO.1) respectively, and performing prokaryotic codon optimization to obtain the sequences. The spliced genes were synthesized by the Synthesis Department of Sangon Biotech (Shanghai) Co., Ltd., and constructed into the pET28TEV vectors, which were named pET28TEV-N730-FRB and pET28TEV-FKBP-C731 respectively. Soluble protein expression was induced at low temperatures, and the target proteins were obtained through affinity purification and molecular sieve purification, which were named N730-FRB (SEQ ID NO: 4) and FKBP-C731 (SEQ ID NO: 5) respectively.

### 1.2 Preparation of crRNA and Activator dsDNA (Cas Protein Cis-Cleavage Substrate)

The crRNA is a screened RNA that efficiently activates the endonuclease activity of the CRISPR/LbCas12a protein, while the CRISPR/LbCas12a activator double-stranded DNA (activator dsDNA, abbreviated as "activator") is the double-stranded DNA corresponding to the crRNA. Both were synthesized by the Synthesis Department of Sangon Biotech (Shanghai) Co., Ltd.

The crRNA and activator dsDNA provided in this example are SEQ ID NO.8, SEQ ID NO.9, and SEQ ID NO.10, with specific details shown in Table 2.

**Table 2 crRNA and Activator dsDNA**

| **SEQ name** | **Name** | **Sequence** |
|---|---|---|
| SEQ NO.8 | crRNA | AAUUUCUACUCUUGUAGAUUUA UCGCAACUUUCUACUGAAUU |
| SEQ NO.9 | activator dsDNA-R | CCGAATTCAGTAGAAAGTTGCGA TAACAAAACTGGCCGTCGTTTTAC AAC |
| SEQ NO.10 | activator dsDNA-F | GTTGTAAAACGACGGCCAGTTTT GTTATCGCAACTTTCTACTGAATT CGG |

This detection uses a final volume of 20 µL as shown in Table 3 and Table 4, but is not limited thereto, including adjustments to the proportions of corresponding components:

**Table 3 Pre-incubation System for Molecular Target and Split LbCas12a**

| **Component** | **Volume** |
|---|---|
| 10× Buffer I | 1 µL |
| N730-FRB (1 µM) | 2 µL |
| FKBP-C731 (1 µM) | 2 µL |
| Rapamycin, its analogs, or control | 0.5 µL |
| H₂O (RNA-free) | Up to 10 µL |

**Table 4 Split CRISPR/Cas12a Fluorescence Detection System**

| **Component** | **Volume** |
|---|---|
| 10× Buffer I | 1 µL |
| Activator (1 µM) | 1 µL |
| crRNA (1 µM) | 1 µL |
| Trans-cleavage reporter (10 µM) | 0.5 µL |
| H₂O (RNA-free) | Up to 10 µL |

wherein, the trans-cleavage reporter is a ssDNA FQ reporter.

The components of 10× Buffer I include 10× HOLMES Buffer I (purchased from Anhui Tolo Biotechnology Company Limited, the same below), with the pH value adjusted from 8.5 to 7.4, and supplemented with NaCl at a final concentration of 100 mM and Tween 20 at a volume ratio of 0.2%.

### 1.3 Specificity of Detection of Rapamycin and Its Analogs by Full-Wavelength Microplate Reader Fluorescence Assay

In the specificity detection case, rapamycin, its analogs (Everolimus, Deforolimus), and controls (Rifamycin, Ampicillin, Kanamycin, Spectinomycin, Chloromycetin, tacrolimus, DMSO) were pre-incubated with the split LbCas12a fusion protein at room temperature for 10 min according to the system in Table 3. Subsequently, the pre-mixed fluorescence detection system from Table 4 was added, and after thorough mixing, the mixture was reacted at 37°C for 5 min.

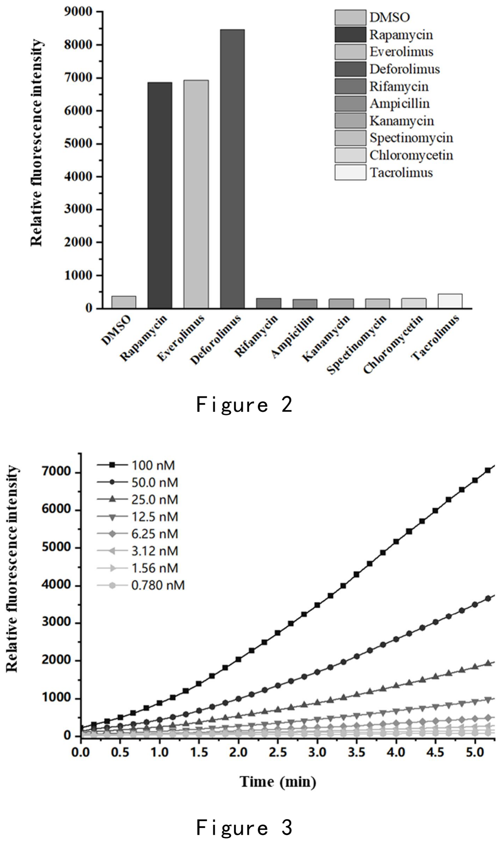

The detection activity of the split CRISPR/LbCas12a detection system was determined by fluorescence detection. A full-wavelength microplate reader was used to measure the fluorescence of the detection reaction and monitor the fluorescence kinetics, with an excitation wavelength of 485 nm and an emission wavelength of 520 nm. Fluorescence values were detected at 5 min after the reaction initiation. This invention enables the specific detection of rapamycin and its analogs, including but not limited to Everolimus and Deforolimus, using the fluorescence-based result determination protocol (see Figure 2).

### 1.4 Sensitivity of Detection of Rapamycin by Full-Wavelength Microplate Reader Fluorescence Assay

In the sensitivity detection case, rapamycin was serially diluted to obtain test samples with concentrations of 100, 50, 25, 12.5, 6.25, 3.12, and 1.56 nM. Each of these samples was pre-incubated with the split LbCas12a fusion protein at room temperature for 10 min according to the system in Table 3. Subsequently, the pre-mixed fluorescence detection system from Table 4 was added, and after thorough mixing, the mixture was reacted at 37°C for 5 min.

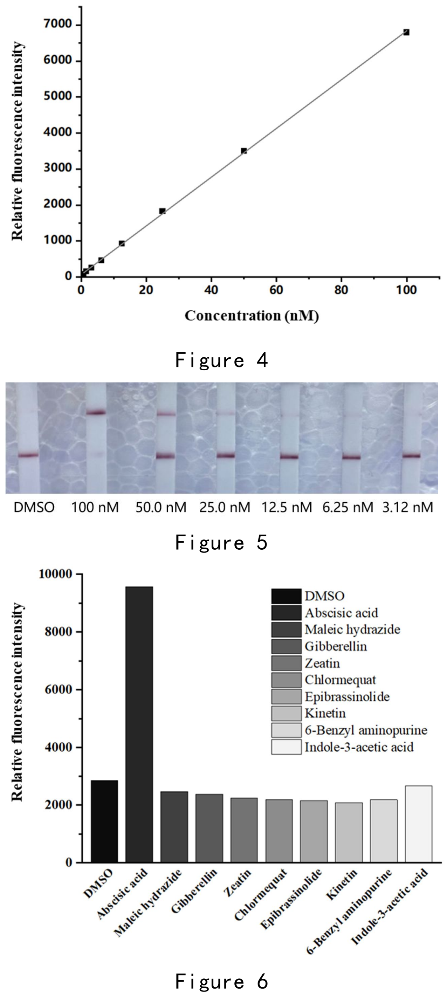

In this example, the detection activity of the split CRISPR/LbCas12a detection system was determined by fluorescence detection. A full-wavelength microplate reader was used to measure the fluorescence of the detection reaction and monitor the fluorescence kinetics, with an excitation wavelength of 485 nm and an emission wavelength of 520 nm. Fluorescence values were detected at 30 min after the reaction initiation (see Figure 3). In this example, the application of the split CRISPR/LbCas12a fluorescence method for detecting rapamycin enabled high-sensitivity detection of rapamycin at a concentration of 1.56 nM (see Figure 4).

### 1.5 Sensitivity of Colloidal Gold Strip Detection for Rapamycin

Based on the results obtained in Example 1.4, the use of the split CRISPR/LbCas12a system demonstrated high sensitivity for rapamycin detection. Therefore, this system was employed for subsequent colloidal gold strip assays.

This detection uses a final volume of 50 µL as shown in Table 5 and Table 6, but is not limited thereto, including adjustments to the proportions of corresponding components:

**Table 5 Pre-incubation System for Target and Split LbCas12a**

| **Component** | **Volume** |
|---|---|
| 10× Buffer I | 2.5 µL |
| N730-FRB (1 µM) | 5 µL |
| FKBP-C731 (1 µM) | 5 µL |
| Target, target analog, or control | 1.25 µL |
| H₂O (RNA-free) | Up to 25 µL |

**Table 6 Split CRISPR/Cas12a Colloidal Gold Detection System**

| **Component** | **Volume** |
|---|---|
| 10× Buffer I | 2.5 µL |
| Activator (1 µM) | 2.5 µL |
| crRNA (1 µM) | 2.5 µL |
| Trans-cleavage reporter (10 µM) | 1.25 µL |
| H₂O (RNA-free) | Up to 25 µL |

wherein, the trans-cleavage reporter is a ssDNA FB reporter.

The components of 10× Buffer I include 10× HOLMES Buffer I, with the pH value adjusted from 8.5 to 7.4, and supplemented with NaCl at a final concentration of 100 mM and Tween 20 at a volume ratio of 0.2%.

In this example, rapamycin was serially diluted to obtain test samples with concentrations of 100, 50, 25, 12.5, 6.25, and 3.12 nM. Each of these samples was pre-incubated with the split LbCas12a fusion protein at room temperature for 10 min according to the system in Table 5. Subsequently, the pre-mixed detection system from Table 6 was added, and after thorough mixing, the mixture was reacted at 37°C for 30 min. Subsequently, the colloidal gold test strip in this example was inserted and incubated at room temperature for 3 min. The detection results of the test strip were judged, photographed and saved, as shown in Figure 5. The results showed that both the split CRISPR/LbCas12a fluorescence method and the colloidal gold test strip could achieve sensitive, rapid and accurate detection of rapamycin.

### Example 2: Rapid and Sensitive Detection of Abscisic Acid (ABA)

### 1.1 Protein Preparation

The gene fragments of ABI and PYL1 in this case were obtained by referencing the ABI and PYL1 gene segments from the NCBI database (amino acid positions 126-424 of accession NC_003075.7 (corresponding to ABI) and amino acid positions 33-210 of accession NP_001331305.1 (corresponding to PYL1)), splicing them with the N730 and C731 segments of the LbCas12a protein (SEQ ID NO.1) respectively, and performing prokaryotic codon optimization to obtain the sequences. The spliced genes were synthesized by the Synthesis Department of Sangon Biotech (Shanghai) Co., Ltd., and constructed into the pET28TEV vectors, which were named pET28TEV-N730-ABI and pET28TEV-PLY1-C731 respectively. Soluble protein expression was induced at low temperatures, and the target proteins were obtained through affinity purification and molecular sieve purification, which were named N730-ABI (SEQ ID NO: 6) and PLY1-C731 (SEQ ID NO: 7) respectively.

### 2.2 Preparation of crRNA and Activator dsDNA

The crRNA is a screened RNA that efficiently activates the endonuclease activity of the CRISPR/LbCas12a protein, while the CRISPR/LbCas12a activator double-stranded DNA (activator dsDNA) is the double-stranded DNA corresponding to the crRNA. Both were synthesized by the Synthesis Department of Sangon Biotech (Shanghai) Co., Ltd.

The crRNA and activator dsDNA provided in this example are SEQ ID NO.8, SEQ ID NO.9, and SEQ ID NO.10, with specific details shown in Table 2.

This detection uses a final volume of 20 µL as shown in Table 7 and Table 8, but is not limited thereto, including adjustments to the proportions of corresponding components:

**Table 7 Pre-incubation System for Molecular Target and Split LbCas12a**

| **Component** | **Volume** |
|---|---|
| 10× Buffer II | 1 µL |
| N730-ABI (1 µM) | 2 µL |
| PYL1-C731 (1 µM) | 2 µL |
| Abscisic acid or control | 0.5 µL |
| H₂O (RNA free) | Up to 10 µL |

**Table 8 Split CRISPR/Cas12a Fluorescence Detection System**

| **Component** | **Volume** |
|---|---|
| 10× Buffer II | 1 µL |
| Activator (1 µM) | 1 µL |
| crRNA (1 µM) | 1 µL |
| Trans-cleavage reporter (10 µM) | 0.5 µL |
| H₂O (RNA-free) | Up to 10 µL |

wherein, the trans-cleavage reporter is a ssDNA FQ reporter.

The components of 10× Buffer II include 10× HOLMES Buffer I, with the pH value adjusted from 8.5 to 8.0, and supplemented with NaCl at a final concentration of 80 mM, NaAc at a final concentration of 20 mM with a pH of 5.5, and Tween 20 at a volume ratio of 0.2%.

### 2.3 Specificity of Detection of ABA by Full-Wavelength Microplate Reader Fluorescence Assay

In the specificity detection case, ABA and controls (Maleic hydrazide, Gibberellin, Zeatin, Chlormequat, Epibrassinolide, Kinetin, 6-Benzyl aminopurine, indole-3-acetic acid, DMSO) were pre-incubated with the split LbCas12a fusion protein at room temperature for 10 min according to the system in Table 7. Subsequently, the pre-mixed fluorescence detection system from Table 8 was added, and after thorough mixing, the mixture was reacted at 37°C for 5 min.

The detection activity of the split CRISPR/LbCas12a detection system was determined by fluorescence detection. A full-wavelength microplate reader was used to measure the fluorescence of the detection reaction and monitor the fluorescence kinetics, with an excitation wavelength of 485 nm and an emission wavelength of 520 nm. Fluorescence values were detected at 5 min after the reaction initiation. This invention enables the specific detection of ABA, using the fluorescence-based result determination protocol (see Figure 6).

### 2.4 Sensitivity of Detection of ABA by Full-Wavelength Microplate Reader Fluorescence Assay

In the sensitivity detection case, ABA was serially diluted to obtain test samples with concentrations of 500, 250, 125, 62.5, 31.2, 15.6, and 7.80 nM. Each of these samples was pre-incubated with the split LbCas12a fusion protein at room temperature for 10 min according to the system in Table 7. Subsequently, the pre-mixed fluorescence detection system from Table 8 was added, and after thorough mixing, the mixture was reacted at 37°C for 5 min.

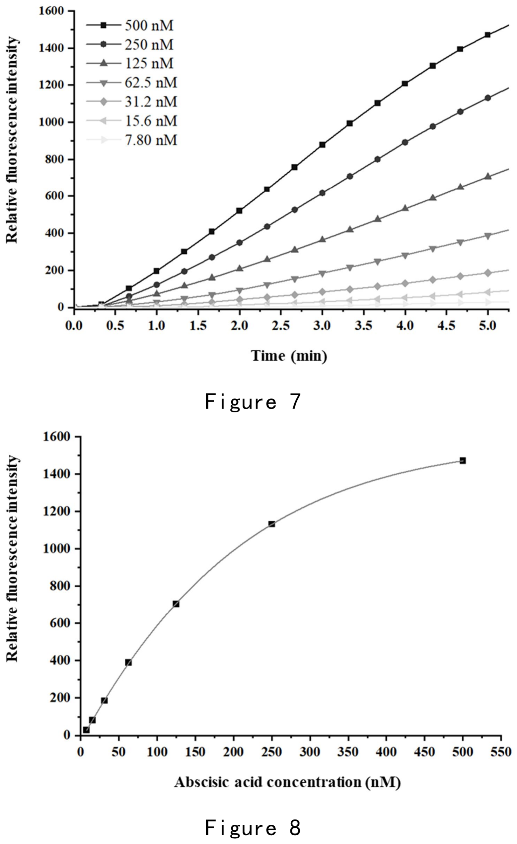

In this example, the detection activity of the split CRISPR/LbCas12a detection system was determined by fluorescence detection. A full-wavelength microplate reader was used to measure the fluorescence of the detection reaction and monitor the fluorescence kinetics, with an excitation wavelength of 485 nm and an emission wavelength of 520 nm. Fluorescence values were detected at 30 min after the reaction initiation (see Figure 7). In this example, the application of the split CRISPR/LbCas12a fluorescence method for detecting ABA enabled high-sensitivity detection of ABA at a concentration of 7.80 nM (see Figure 8).

### Example 3 Experimental Method and Results for Rapamycin Detection Using Split Cas12a

### 3.1 Basic Composition of the Reaction System

**Table 9 Basic Composition of the Reaction System**

| Component | Volume (µL) | Final concentration |
|---|---|---|
| 10× HOLMES buffer 1 | 2 | 1× |
| Mix split Cas12a* | 2 | 100 nM |
| crRNA | 0.1 | 50 nM |
| Activator** | 0.1 | 50 nM |
| Trans-cleavage reporter | 0.5 | 250 nM |
| Rapa | 0.5 | / |
| H₂O | Up to 20 | / |

| | | |
|---|---|---|
| * The Mix split Cas12a in Tables 9 and 10 of this example refers to the mixture of N730-FRB (1 µM) and FKBP-C731 (1 µM) used in Example 1. ** The Activator in Tables 9 and 10 of this example uses only activator dsDNA-R (SEQ NO.9). | | |

### 3.2 System Optimization - Reaction pH

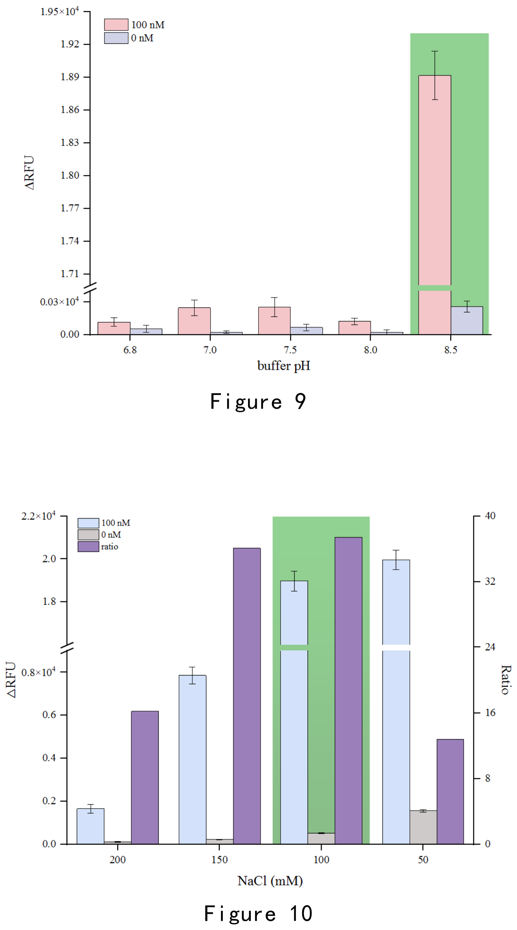

Based on the isoelectric points of the two split Cas12a fragment proteins (N730-FRB and FKBP-C731, identical to those described in Example 1 above), which are 6.623 and 9.106 respectively, the reaction pH of the HOLMES buffer was screened at pH values of 6.8, 7.0, 7.5, 8.0, and 8.5. The experimental results showed that the optimal reaction pH for this system is 8.5 (see Figure 9, and in subsequent experiments, unless otherwise specified, the buffers of the reaction system all use a pH of 8.5), and ΔRFU represents the relative fluorescence intensity after 30 minutes of reaction.

### 3.3 System Optimization - Reaction Additives

After determining the reaction pH, considering the relatively high background signal, different additives were designed to effectively suppress the background signal. Several concentrations of NaCl (final concentrations ranging from 50-200 mM) were tested. The detection results showed that the combination of NaCl at a final concentration of 100 mM and Tween 20 at a final concentration of 0.2% (V/V) achieved the best effect (see Figure 10, and in subsequent experiments, unless otherwise specified, the additives of the buffers of the reaction system all use the combination of NaCl at a final concentration of 100 mM and Tween 20 at a final concentration of 0.2% (V/V)), and the ratio of ΔRFU (100 nM rapamycin) to ΔRFU (0 nM rapamycin) was the highest (purple).

### 3.4 Specificity of the Reaction System

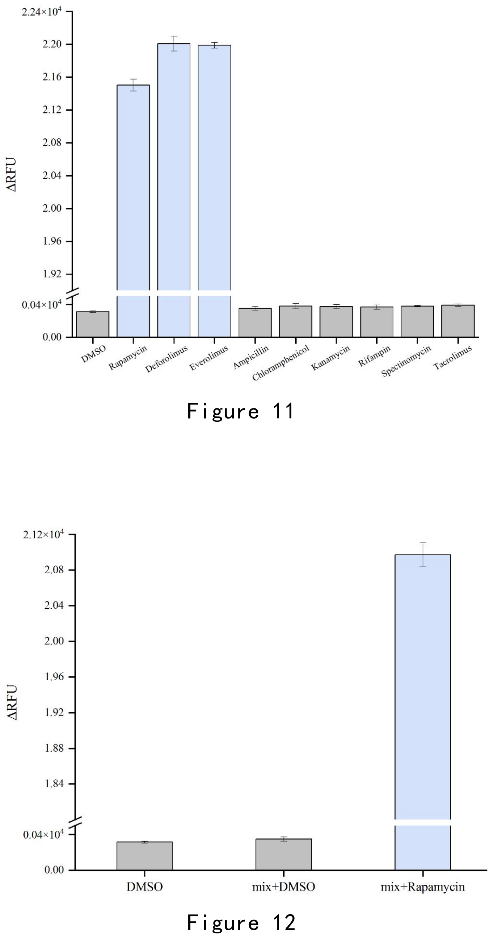

A comparison was made between 100 nM different types of small molecules and rapamycin, wherein the small molecules include rapamycin analogs (Deforolimus and Everolimus), the antagonist Tacrolimus, and other types of antibiotics such as Ampicillin, Chloramphenicol, Kanamycin, Rifampin, and Spectinomycin. The experimental results showed that the system only recognized rapamycin and its analogs, with fluorescent signals generated at a concentration of 100 nM (Figure 11). Furthermore, rapamycin was mixed with other small molecules excluding rapamycin analogs, with each concentration still set at 100 nM, and the resulting mixtures were subjected to detection. The detection results, as shown in Figure 12, indicated that the system could still specifically recognize rapamycin from the mixtures and thus exhibit fluorescent signals.

### 3.5 Sensitivity of the Reaction System

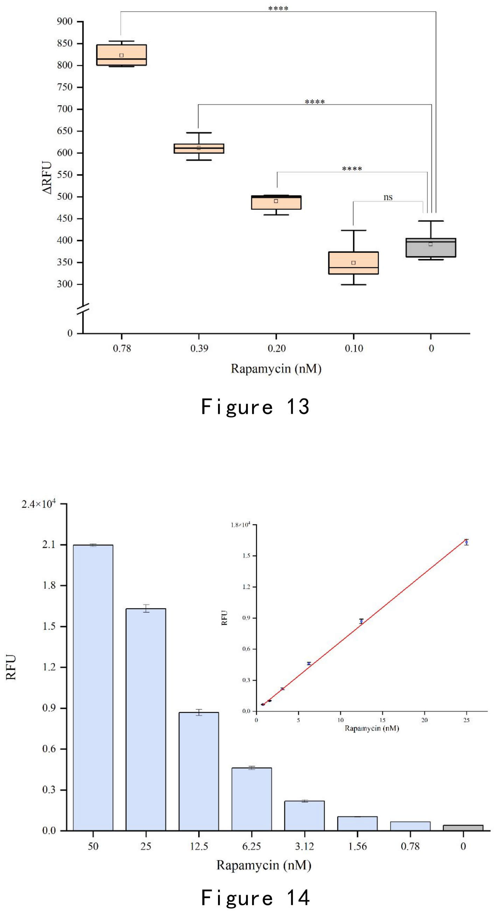

Rapamycin was serially diluted starting from 100 nM, with the second concentration being 50 nM, and so on until the 11th gradient, where the concentration of rapamycin was approximately 0.10 nM. The detection results showed that when the concentration of rapamycin was about 0.20 nM, the reaction system exhibited an extremely significant difference from the negative control (Figure 13), indicating that the minimum detection limit of this system could reach 0.20 nM.

### 3.6 Linearity of the Reaction System

Rapamycin was diluted according to the above method. The results showed that when the concentration of rapamycin was in the range of 0-25 nM, the reaction system had a good linear relationship within 5-10 minutes (Figure 14, R² > 0.99).

### 3.7 Processing of Commercial Serum Samples

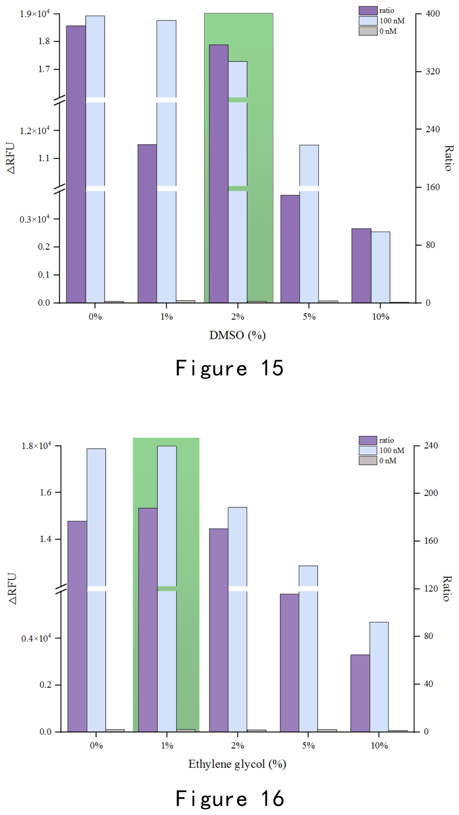

First, an appropriate amount of commercial serum was added to the outer extraction bottle, followed by slow dropwise addition of an equal volume of DMSO. The extraction column was capped, and the mixture was immediately vortexed for 10-20 seconds. Subsequently, pressure was applied to allow the liquid to move upward along the extraction column, and 2 µL of the liquid was directly pipetted for detection. The composition of the reaction system at this point is shown in Table 10. The extraction column was purchased according to another patent (CN 110327658 A). The final concentration of DMSO in the reaction system did not exceed 10% (V/V), with the optimal concentration being 2% (V/V) (see Figure 15, and in subsequent experiments, unless otherwise specified, the final concentration of DMSO used in sample processing within the reaction system is 2% (V/V)).

**Table 10 Basic Composition of the Reaction System in Serum Matrix**

| Component | Volume (µL) | Final concentration |
|---|---|---|
| 10×HOLMES buffer 1 | 2 | 1× |
| Mix split Cas12a | 2 | 100 nM |
| crRNA | 0.1 | 50 nM |
| Activator | 0.1 | 50 nM |
| Trans-cleavage reporter | 0.5 | 250 nM |
| NaCl | 2 | 0.5 mM |
| Tween 20 | 2 | 0.1% |
| Sample | 2 | / |
| H₂O | To 20 | / |

### 3.8 Processing of EDTA Bovine Whole Blood Samples

An appropriate amount of EDTA bovine whole blood sample was placed into a 1.5 mL EP tube. An equal volume of sample pretreatment solution (SPR) was slowly added along the tube wall, and the mixture was immediately vortexed for 10-20 seconds. It was then centrifuged at 13,000 rpm for 10 minutes at room temperature, and 2 µL of the liquid was taken for detection. The detection system is shown in Table 10. SPR = 70% (V/V) DMSO + 30%(V/V) ethylene glycol (EG). The proportion of EG in the reaction system does not exceed 10% (V/V), with the optimal proportion being 1% (V/V), which can be adjusted within the range of 0-2% (V/V) (as shown in Figure 16).

### 3.9 Sensitivity and Linear Range for Detecting Commercial Serum Samples

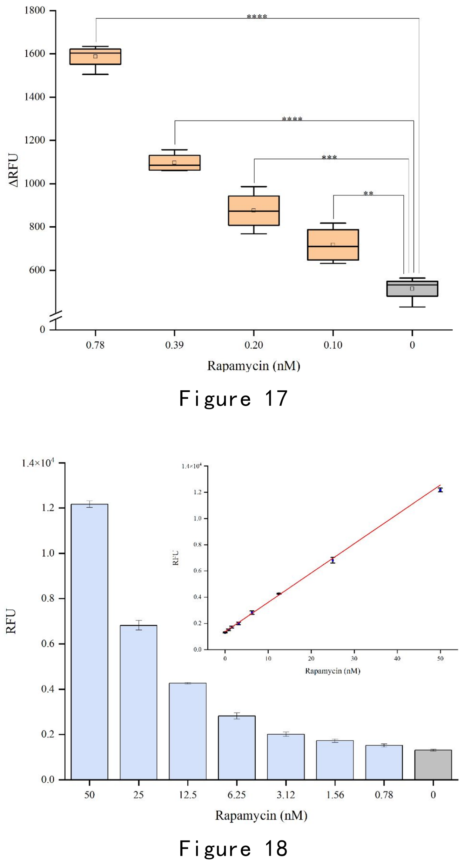

When the concentration of rapamycin was 0.1 nM, there was a significant difference compared with the negative control (Figure 17), indicating that the minimum detection limit for serum sample detection could reach 0.1 nM. When the concentration of rapamycin in serum samples was in the range of 0-50 nM, a good linear relationship was observed within 5-10 minutes of reaction (Figure 18, R² > 0.99).

### 3.10 Sensitivity and Linear Range for Detecting EDTA Bovine Whole Blood Samples

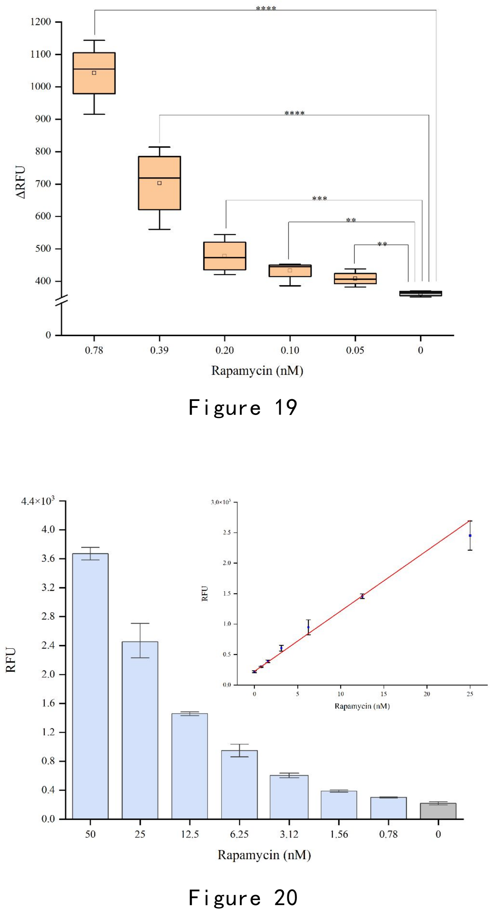

When the concentration of rapamycin in EDTA bovine whole blood samples was 0.05 nM, there was still a significant difference compared with the negative control, so the minimum detection limit of this system for this sample type was 0.05 nM (Figure 19). When the concentration of rapamycin in EDTA bovine whole blood samples was in the range of 0-25 nM, a good linear range was achieved within 5-10 minutes of reaction (Figure 20, R² > 0.99).

### Sequencing information

SEQ ID NO: 1 LbCas12a 
MSKLEKFTNCYSLSKTLRFKAIPVGKTQENIDNKRLLVEDEKRAEDYKG
VKKLLDRYYLSFINDVLHSIKLKNLNNYISLFRKKTRTEKENKELENLEINLR
KEIAKAFKGNEGYKSLFKKDIIETILPEFLDDKDEIALVNSFNGFTTAFTGFFD
NRENMFSEEAKSTSIAFRCINENLTRYISNMDIFEKVDAIFDKHEVQEIKEKIL
NSDYDVEDFFEGEFFNFVLTQEGIDVYNAIIGGFVTESGEKIKGLNEYINLYN
QKTKQKLPKFKPLYKQVLSDRESLSFYGEGYTSDEEVLEVFRNTLNKNSEIFS
SIKKLEKLFKNFDEYSSAGIFVKNGPAISTISKDIFGEWNVIRDKWNAEYDDIH
LKKKAVVTEKYEDDRRKSFKKIGSFSLEQLQEYADADLSVVEKLKEIIIQKVD
EIYKVYGSSEKLFDADFVLEKSLKKNDAVVAIMKDLLDSVKSFENYIKAFFG
EGKETNRDESFYGDFVLAYDILLKVDHIYDAIRNYVTQKPYSKDKFKLYFQN
PQFMGGWDKDKETDYRATILRYGSKYYLAIMDKKYAKCLQKIDKDDVNGN
YEKINYKLLPGPNKMLPKVFFSKKWMAYYNPSEDIQKIYKNGTFKKGDMFN
LNDCHKLIDFFKDSISRYPKWSNAYDFNFSETEKYKDIAGFYREVEEQGYKV
SFESASKKEVDKLVEEGKLYMFQIYNKDFSDKSHGTPNLHTMYFKLLFDENN
HGQIRLSGGAELFMRRASLKKEELVVHPANSPIANKNPDNPKKTTTLSYDVY
KDKRFSEDQYELHIPIAINKCPKNIFKINTEVRVLLKHDDNPYVIGIDRGERNL
LYIVVVDGKGNIVEQYSLNEIINNFNGIRIKTDYHSLLDKKEKERFEARQNWT
SIENIKELKAGYISQVVHKICELVEKYDAVIALEDLNSGFKNSRVKVEKQVYQ
KFEKMLIDKLNYMVDKKSNPCATGGALKGYQITNKFESFKSMSTQNGFIFYI
PAWLTSKIDPSTGFVNLLKTKYTSIADSKKFISSFDRIMYVPEEDLFEFALDYK
NFSRTDADYIKKWKLYSYGNRIRIFRNPKKNNVFDWEEVCLTSAYKELFNKY
GINYQQGDIRALLCEQSDKAFYSSFMALMSLMLQMRNSITGRTDVDFLISPV
KNSDGIFYDSRNYEAQENAILPKNADANGAYNIARKVLWAIGQFKKAEDEK
LDKVKIAISNKEWLEYAQTSVKH*
SEQ ID NO: 2 Linker peptide
   EFGGSGSSGGSGGSGGSG
SEQ ID NO: 3 Linker peptide
   GGSGGSGGGSGSGSGGGT
SEQ ID NO: 4 N730-FRB (N-terminal fragment of LbCas12a (Cas₁₋₇₃₀ fragment) - linker peptide - rapamycin receptor) 
MSKLEKFTNCYSLSKTLRFKAIPVGKTQENIDNKRLLVEDEKRAEDYKG
VKKLLDRYYLSFINDVLHSIKLKNLNNYISLFRKKTRTEKENKELENLEINLR
KEIAKAFKGNEGYKSLFKKDIIETILPEFLDDKDEIALVNSFNGFTTAFTGFFD
NRENMFSEEAKSTSIAFRCINENLTRYISNMDIFEKVDAIFDKHEVQEIKEKIL
NSDYDVEDFFEGEFFNFVLTQEGIDVYNAIIGGFVTESGEKIKGLNEYINLYN
QKTKQKLPKFKPLYKQVLSDRESLSFYGEGYTSDEEVLEVFRNTLNKNSEIFS
SIKKLEKLFKNFDEYSSAGIFVKNGPAISTISKDIFGEWNVIRDKWNAEYDDIH
LKKKAVVTEKYEDDRRKSFKKIGSFSLEQLQEYADADLSVVEKLKEIIIQKVD
EIYKVYGSSEKLFDADFVLEKSLKKNDAVVAIMKDLLDSVKSFENYIKAFFG
EGKETNRDESFYGDFVLAYDILLKVDHIYDAIRNYVTQKPYSKDKFKLYFQN
PQFMGGWDKDKETDYRATILRYGSKYYLAIMDKKYAKCLQKIDKDDVNGN
YEKINYKLLPGPNKMLPKVFFSKKWMAYYNPSEDIQKIYKNGTFKKGDMFN
LNDCHKLIDFFKDSISRYPKWSNAYDFNFSETEKYKDIAGFYREVEEQGYKV
SFESASKKEVDKLVEEGKLYMFQIYNKDFSDKSHGTPNLHTMYFKLLFDEEF
GGSGSSGGSGGSGGSGILWHEMWHEGLEEASRLYFGERNVKGMFEVLEPLH
AMMERGPQTLKETSFNQAYGRDLMEAQEWCRKYMKSGNVKDLTQAWDLY
YHVFRRISKGGSGGSGGGSGSGSGG*
SEQ ID NO: 5 FKBP-C731 (Rapamycin receptor - linker peptide - C-terminal fragment of LbCas12a (Cas₇₃₁₋₁₂₂₈ fragment)) 
MGGSGSSGGSGGSGGSGMGVQVETISPGDGRTFPKRGQTCVVHYTGML
EDGKKFDSSRDRNKPFKFMLGKQEVIRGWEEGVAQMSVGQRAKLTISPDYA
YGATGHPGIIPPHATLVFDVELLKLEGGSGGSGGGSGSGSGGGTNNHGQIRLS
 
GGAELFMRRASLKKEELVVHPANSPIANKNPDNPKKTTTLSYDVYKDKRFSE
DQYELHIPIAINKCPKNIFKINTEVRVLLKHDDNPYVIGIDRGERNLLYIVVVD
GKGNIVEQYSLNEIINNFNGIRIKTDYHSLLDKKEKERFEARQNWTSIENIKEL
KAGYISQVVHKICELVEKYDAVIALEDLNSGFKNSRVKVEKQVYQKFEKMLI
DKLNYMVDKKSNPCATGGALKGYQITNKFESFKSMSTQNGFIFYIPAWLTSK
IDPSTGFVNLLKTKYTSIADSKKFISSFDRIMYVPEEDLFEFALDYKNFSRTDA
DYIKKWKLYSYGNRIRIFRNPKKNNVFDWEEVCLTSAYKELFNKYGINYQQ
GDIRALLCEQSDKAFYSSFMALMSLMLQMRNSITGRTDVDFLISPVKNSDGIF
YDSRNYEAQENAILPKNADANGAYNIARKVLWAIGQFKKAEDEKLDKVKIA
ISNKEWLEYAQTSVKH*
SEQ ID NO: 6 N730-ABI (N-terminus of LbCas12a (N730) + linker peptide + abscisic acid receptor) 
MSKLEKFTNCYSLSKTLRFKAIPVGKTQENIDNKRLLVEDEKRAEDYKG
VKKLLDRYYLSFINDVLHSIKLKNLNNYISLFRKKTRTEKENKELENLEINLR
KEIAKAFKGNEGYKSLFKKDIIETILPEFLDDKDEIALVNSFNGFTTAFTGFFD
NRENMFSEEAKSTSIAFRCINENLTRYISNMDIFEKVDAIFDKHEVQEIKEKIL
NSDYDVEDFFEGEFFNFVLTQEGIDVYNAIIGGFVTESGEKIKGLNEYINLYN
QKTKQKLPKFKPLYKQVLSDRESLSFYGEGYTSDEEVLEVFRNTLNKNSEIFS
SIKKLEKLFKNFDEYSSAGIFVKNGPAISTISKDIFGEWNVIRDKWNAEYDDIH
LKKKAVVTEKYEDDRRKSFKKIGSFSLEQLQEYADADLSVVEKLKEIIIQKVD
EIYKVYGSSEKLFDADFVLEKSLKKNDAVVAIMKDLLDSVKSFENYIKAFFG
EGKETNRDESFYGDFVLAYDILLKVDHIYDAIRNYVTQKPYSKDKFKLYFQN
PQFMGGWDKDKETDYRATILRYGSKYYLAIMDKKYAKCLQKIDKDDVNGN
YEKINYKLLPGPNKMLPKVFFSKKWMAYYNPSEDIQKIYKNGTFKKGDMFN
LNDCHKLIDFFKDSISRYPKWSNAYDFNFSETEKYKDIAGFYREVEEQGYKV
SFESASKKEVDKLVEEGKLYMFQIYNKDFSDKSHGTPNLHTMYFKLLFDEEF
GGSGSSGGSGGSGGSGVPLYGFTSICGRRPEMEAAVSTIPRFLQSSSGSMLDG
RFDPQSAAHFFGVYDGHGGSQVANYCRERMHLALAEEIAKEKPMLCDGDT
WLEKWKKALFNSFLRVDSEIESVAPETVGSTSVVAVVFPSHIFVANCGDSRA
 
VLCRGKTALPLSVDHKPDREDEAARIEAAGGKVIQWNGARVFGVLAMSRSI
GDRYLKPSIIPDPEVTAVKRVKEDDCLILASDGVWDVMTDEEACEMARKRIL
LWHKKNAVAGDASLLADERRKEGKDPAAMSAAEYLSKLAIQRGSKDNISV
VVVDLKGGSGGSGGGSGSGSGG*
SEQ ID NO: 7 PLY1-C731 (Abscisic acid receptor + linker peptide + C-terminus of LbCas12a (C731)) 
MGGSGSSGGSGGSGGSGTQDEFTQLSQSIAEFHTYQLGNGRCSSLLAQR
IHAPPETVWSVVRRFDRPQIYKHFIKSCNVSEDFEMRVGCTRDVNVISGLPAN
TSRERLDLLDDDRRVTGFSITGGEHRLRNYKSVTTVHRFEKEEEEERIWTVV
LESYVVDVPEGNSEEDTRLFADTVIRLNLQKLASITEAMNGGSGGSGGGSGS
GSGGGTNNHGQIRLSGGAELFMRRASLKKEELVVHPANSPIANKNPDNPKKT
TTLSYDVYKDKRFSEDQYELHIPIAINKCPKNIFKINTEVRVLLKHDDNPYVIG
IDRGERNLLYIVVVDGKGNIVEQYSLNEIINNFNGIRIKTDYHSLLDKKEKERF
EARQNWTSIENIKELKAGYISQVVHKICELVEKYDAVIALEDLNSGFKNSRV
KVEKQVYQKFEKMLIDKLNYMVDKKSNPCATGGALKGYQITNKFESFKSMS
TQNGFIFYIPAWLTSKIDPSTGFVNLLKTKYTSIADSKKFISSFDRIMYVPEEDL
FEFALDYKNFSRTDADYIKKWKLYSYGNRIRIFRNPKKNNVFDWEEVCLTSA
YKELFNKYGINYQQGDIRALLCEQSDKAFYSSFMALMSLMLQMRNSITGRT
DVDFLISPVKNSDGIFYDSRNYEAQENAILPKNADANGAYNIARKVLWAIGQ
FKKAEDEKLDKVKIAISNKEWLEYAQTSVKH*
SEQ NO.8 crRNA
   AAUUUCUACUCUUGUAGAUUUAUCGCAACUUUCUACUGAAUU
SEQ NO.9 activator dsDNA-R 
CCGAATTCAGTAGAAAGTTGCGATAACAAAACTGGCCGTCGTTTTAC
AAC
SEQ NO.10 activator dsDNA-F

GTTGTAAAACGACGGCCAGTTTTGTTATCGCAACTTTCTACTGAATTC
GG

All literatures mentioned in the present invention are incorporated herein by reference, as though each one is individually incorporated by reference. In addition, it should be understood that, after reading the above teachings of the present invention, those skilled in the art can make various changes or modifications to the present invention, these equivalents also fall within the scope as defined in the appended claims of the present application.

## Claims

1. A detection system for detecting a non-nucleic acid target, comprising:
(a1) a first fusion protein or a first binding protein, wherein the structure of the first fusion protein or the first binding protein is as set forth in Formula (I):
Z1-L1-A1 (I);
(a2) a second fusion protein or a second binding protein, wherein the structure of the second fusion protein or the second binding protein is as set forth in Formula (II):
A2-L2-Z2 (II);
wherein,
Z1 is a N-terminal fragment of a Cas protein;
L1 is a first linker peptide;
A1 is a first target receptor;
A2 is a second target receptor;
L2 is a second linker peptide;
Z2 is a C-terminal fragment of a Cas protein;
each "-" independently represents a linker peptide, a peptide bond, or a non-peptide bond;
(b) a Cas protein cis-cleavage substrate or a Cas protein cis-cleavage reporter; and
(c) a guide RNA, wherein the guide RNA guides the Cas protein to specifically bind to the Cas protein cis-cleavage substrate or the Cas protein cis-cleavage reporter.

2. The detection system according to claim 1, wherein the Cas protein cis-cleavage substrate is a component of the Cas protein cis-cleavage reporter.

3. The detection system according to claim 1, wherein the detection system for detecting a non-nucleic acid target further comprises:
(d) a Cas protein trans-cleavage reporter, wherein the Cas protein trans-cleavage reporter has a single-stranded nucleic acid molecule that does not hybridize with the guide sequence of the guide RNA of the Cas protein.

4. A kit for detecting a non-nucleic acid target, comprising:
i) a first container and a first fusion protein or a first binding protein, and a second fusion protein or a second binding protein located within the first container; the structure of the first fusion protein or the first binding protein is as set forth in Formula I:
Z1-L1-A1 (I);
the structure of the second fusion protein or the second binding protein is as set forth in Formula II:
A2-L2-Z2 (II);
wherein,
Z1 is a N-terminal fragment of a Cas protein;
L1 is a first linker peptide;
A1 is a first target receptor;
A2 is a second target receptor;
L2 is a second linker peptide;
Z2 is a C-terminal fragment of a Cas protein;
each "-" independently represents a linker peptide, a peptide bond, or a non-peptide bond;
ii) a second container and a Cas protein cis-cleavage substrate or a Cas protein cis-cleavage reporter located within the second container;
iii) an optional third container and a Cas protein trans-cleavage reporter located within the third container;
iv) a fourth container and a guide RNA located within the fourth container;
v) an optional fifth container and a buffer located within the fifth container.

5. A method for detecting a non-nucleic acid target, comprising the steps of:
providing the detection system for detecting a non-nucleic acid target according to claim 1, wherein the detection system further comprises a sample to be detected; and detecting the signal emitted by the detection system,
wherein, if a signal emitted by the detection system is detected, it indicates that the non-nucleic acid target is present in the sample; and if no signal emitted by the detection system is detected, it indicates that the non-nucleic acid target is not present in the sample.

6. A composition or a product combination, comprising:
(a1) a first fusion protein or a first binding protein, the structure of which is as set forth in Formula I:
Z1-L1-A1 (I);
(a2) a second fusion protein or a second binding protein, the structure of which is as set forth in Formula II:
A2-L2-Z2 (II);
wherein,
Z1 is a N-terminal fragment of a Cas protein;
L1 is a first linker peptide;
A1 is a first target receptor;
A2 is a second target receptor;
L2 is a second linker peptide;
Z2 is a C-terminal fragment of a Cas protein;
each "-" independently represents a linker peptide, a peptide bond, or a non-peptide bond;
(b) a Cas protein cis-cleavage substrate or a Cas protein cis-cleavage reporter; and
(c) a guide RNA, wherein the guide RNA guides the Cas protein to specifically bind to the Cas protein cis-cleavage substrate or the Cas protein cis-cleavage reporter.

7. Use of a composition or a product combination, wherein the composition or the product combination is according to claim 6, **characterized in that** the use of the composition or the product combination is for preparing a reagent, kit or system for detecting a non-nucleic acid target, or for a method of detecting a non-nucleic acid target.

8. A method for preparing a composition, product combination, reagent, kit, or system for detecting a non-nucleic acid target, comprising the steps of:
obtaining a first fusion protein or a first binding protein;
obtaining a second fusion protein or a second binding protein;
obtaining a Cas protein cis-cleavage substrate;
obtaining a guide RNA;
the structure of the first fusion protein or the first binding protein is as set forth in Formula I:
Z1-L1-A1 (I);
the structure of the second fusion protein or the second binding protein is as set forth in Formula II:
A2-L2-Z2 (II);
wherein,
Z1 is a N-terminal fragment of a Cas protein;
L1 is a first linker peptide;
A1 is a first target receptor;
A2 is a second target receptor;
L2 is a second linker peptide;
Z2 is a C-terminal fragment of a Cas protein;
each "-" independently represents a linker peptide, a peptide bond, or a non-peptide bond.

9. Use of a split protein of a Cas protein, a Cas protein cis-cleavage substrate, a Cas protein cis-cleavage reporter, a guide RNA, a Cas protein trans-cleavage reporter, or a buffer, for preparing a composition, product combination, reagent, kit, or system for detecting a non-nucleic acid target, or for a method of detecting a non-nucleic acid target.

10. A reagent, comprising:
a first fusion protein or a first binding protein;
a second fusion protein or a second binding protein;
a Cas protein cis-cleavage substrate or a Cas protein cis-cleavage reporter;
a guide RNA, wherein the guide RNA can guide the Cas protein to bind to the Cas protein cis-cleavage substrate; and
a buffer;
the structure of the first fusion protein or the first binding protein is as set forth in Formula I:
Z1-L1-A1 (I);
the structure of the second fusion protein or the second binding protein is as set forth in Formula II:
A2-L2-Z2 (II);
wherein,
Z1 is a N-terminal fragment of a Cas protein;
L1 is a first linker peptide;
A1 is a first target receptor;
A2 is a second target receptor;
L2 is a second linker peptide;
Z2 is a C-terminal fragment of a Cas protein;
each "-" independently represents a linker peptide, a peptide bond, or a non-peptide bond.
